Europäisches Patentamt
European Patent Office
Office européen des brevets

(11) Veröffentlichungsnummer: **0 000 488**
**A1**

# EUROPÄISCHE PATENTANMELDUNG

(21) Anmeldenummer: 78100327.2

(22) Anmeldetag: 07.07.78

(51) Int. Cl.²: C 07 D 311/22, C 07 D 311/30, C 07 D 311/94, A 61 K 31/35

(30) Priorität: 15.07.77 LU 77786

(43) Veröffentlichungstag der Anmeldung: 07.02.79 Bulletin 79/3

(84) Benannte Vertragsstaaten: BE CH DE FR GB NL SE

(71) Anmelder: CIBA-GEIGY AG
Patentabteilung Postfach
CH-4002 Basel. (CH)

(72) Erfinder: Haas, Georges, Dr.
Im Rehwechsel 24
CH-4102 Binningen (CH)

(72) Erfinder: Rossi, Alberto, Dr.
Bündtenweg 30
CH-4104 Oberwil. (CH)

(72) Erfinder: Jaeggi, Knut A., Dr.
General Guisan-Strasse 44
CH-4054 Basel. (CH)

(72) Erfinder: Sele, Alex
Langmattstrasse 14
CH-4132 Muttenz. (CH)

(74) Vertreter: Zumstein sen., Fritz, Dr. et al
Bräuhausstrasse 4
D-8000 München 2. (DE)

(54) **3-Oxaloamino-4-oxo-4H-1-benzopyranderivaten, Verfahren zu deren Herstellung und deren pharmazeutischen Verwendung.**

(57) Verfahren zur Herstellung neuer 3-Oxaloamino-4-oxo-4H-1-benzopyranderivate

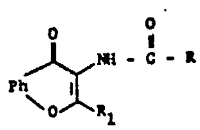

(I),

worin Ph gegebenenfalls substituiertes 1,2-Phenylen darstellt, R gegebenenfalls verestertes oder amidiertes Carboxy darstellt und $R_1$ Wasserstoff oder einen gegebenenfalls substituierten Kohlenwasserstoffrest bedeutet, in freier Form oder in Salzform mit antiallergischer Wirkung.

EP 0 000 488 A1

4-11247/+

BEZEICHNUNG GEÄNDERT
siehe Titelseite

Verfahren zur Herstellung neuer Benzopyranderivate

Die vorliegende Erfindung betrifft neue Benzopyranderivate, insbesondere 3-Oxaloamino-4-oxo-4H-1-benzopyranderivate der Formel I

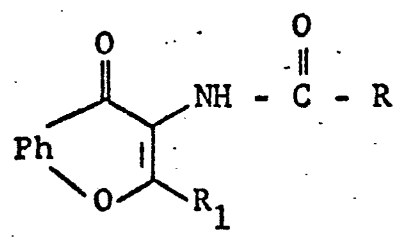

(I) ,

worin Ph gegebenenfalls substituiertes 1,2-Phenylen darstellt, R gegebenenfalls verestertes oder amidiertes Carboxy darstellt, und $R_1$ Wasserstoff oder einen gegebenenfalls substituierten Kohlenwasserstoffrest bedeutet, in freier Form oder in Salzform, Verfahren zur Herstellung derselben, diese enthaltende pharmazeutische Präparate und die Verwendung von Verbindungen der Formel I als Pharmazeutikum oder zur Herstellung pharmazeutischer Präparate.

Gegebenenfalls substituiertes 1,2-Phenylen kann ein- oder mehrfach substituiert sein, wobei als Substituenten beispielsweise aliphatische Reste, wie Niederalkyl oder an zwei benachbarete C-Atome gebundenes Niederalkylen, Acylreste, wie

Niederalkanoyl, gegebenenfalls veräthertes oder verestertes Hydroxy, wie Niederalkoxy, Hydroxyniederalkoxy oder an zwei benachbarte C-Atome gebundenes Niederalkylendioxy oder Halogen, und Trifluormethyl in Betracht kommen.

Gegebenenfalls verestertes oder amidiertes Carboxy ist beispielsweise mit einem Alkohol aliphatischen Charakters verestertes Carboxy, gegebenenfalls durch mindestens einen gegebenenfalls substituierten Aryl- oder gegebenenfalls heteroanalogen Kohlenwasserstoffrest aliphatischen Charakters, Hydroxy oder primäres Amino substituiertes Carbamyl oder vor allem freies Carboxy.

Ein Alkohol aliphatischen Charakters ist ein Alkohol, dessen mit der Hydroxygruppe verbundenes C-Atom nicht Glied eines aromatischen Systems ist, beispielsweise ein gegebenenfalls durch gegebenenfalls substituiertes Phenyl substituiertes Niederalkanol, oder ein cycloaliphatischer Alkohol, z.B. ein 5- bis 8-gliedriges Cycloalkanol. Als Beispiele für mit einem gegebenenfalls substituierten Alkohol aliphatischen Charakters verestertes Carboxy seien genannt: Niederalkoxycarbonyl, z.B. Methoxy-, Aethoxy-, Propoxy-, Isopropoxy- und Butoxycarbonyl, im Phenylteil gegebenenfalls substituiertes Phenylniederalkoxy-, vor allem α- und β-Phenylniederalkoxycarbonyl, z.B. gegebenenfalls substituiertes Benzyloxy- und α- sowie β-Phenäthoxycarbonyl, und 5- bis 8-gliedriges Cycloalkoxycarbonyl, z.B. Cyclopentyloxy-, Cyclohexyloxy- und Cycloheptyloxycarbonyl.

In einem gegebenenfalls substituierten, gegebenenfalls heteroanalogen Kohlenwasserstoffrest aliphatischen Charakters als Substituenten einer Carbamylgruppe geht die freie Valenz von einem nichtaromatischen C-Atom aus. Ein-

solcher Rest ist beispielsweise Niederalkyl oder Niederalkenyl, das, z.B. durch gegebenenfalls substituiertes Phenyl, substituiert sein kann, oder z.B. 5- bis 8-gliedriges Cycloalkyl, wie Cyclohexyl, oder gegebenenfalls niederalkyliertes, gegebenenfalls monooxa-, -aza- oder -thianaloges 4- bis 7-gliedriges Alkylen, beispielsweise Tetra- oder Pentamethylen oder 3-Oxa-, 3-Aza- oder 3-Thiapentamethylen. Als Beispiele für durch mindestens einen solchen Rest substituiertes Carbamyl seien genannt: Mono- oder Diniederalkylcarbamyl, z.B. N-Methyl-, N-Aethyl- oder N,N-Diäthylcarbamyl, im Phenylteil gegebenenfalls substituiertes Phenylniederalkylcarbamyl, wie N-Benzyl- oder N-(1- oder 2-Phenäthyl)-carbamyl, oder Pyrrolidino-, Piperidino-, Morpholino-, Thiomorpholino-, Piperazino- oder 4-Niederalkyl-, z.B. 4-Methylpiperazinocarbonyl.

Ein gegebenenfalls substituierter Kohlenwasserstoffrest $R_1$ ist beispielsweise ein gegebenenfalls substituierter Kohlenwasserstoffrest aliphatischen Charakters oder ein gegebenenfalls substituierter aromatischer Kohlenwasserstoffrest.

In einem gegebenenfalls substituierten Kohlenwasserstoffrest aliphatischen Charakters geht die freie Valenz von einem nichtaromatischen C-Atom aus. Ein solcher Rest ist beispielsweise ein unsubstituierter oder in zweiter Linie durch gegebenenfalls substituiertes Phenyl substituierter aliphatischer Kohlenwasserstoffrest, z.B. Niederalkylrest, oder ferner ein cycloaliphatischer Kohlenwasserstoffrest, wie 5- bis 8-gliedriges Cycloalkyl oder Cycloalkenyl, z.B. 1-Cycloalkenyl. Als Beispiele für solche Reste sind insbesondere zu nennen: Methyl, Aethyl, Isopropyl und Butyl, ferner Benzyl, Cyclopentyl, Cyclohexyl und Cycloheptyl.

Ein gegebenenfalls substituierter aromatischer Kohlenwasserstoffrest (Arylrest) ist beispielsweise gegebenenfalls substituiertes Phenyl.

Vor- und nachstehend gilt:

Mit "nieder" bezeichnete organische Verbindungen oder Reste weisen bis zu 7, vor allem bis zu 4, C-Atome auf und können geradkettig oder verzweigt sein.

Gegebenenfalls substituiertes Phenyl sowie Phenyl in gegebenenfalls substituiertem Phenylniederalkyl und Phenylniederalkoxy ist beispielsweise gegebenenfalls ein- oder mehrfach, substituiertes Phenyl, wobei als Substituenten vor allem Niederalkyl, Niederalkoxy und Halogen, z.B. die nachstehend genannten, sowie Trifluormethyl in Betracht kommen, wie Phenyl, o-, m- oder p-Tolyl, o-, m- oder p-Anisyl, o-, m- oder p-Chlorphenyl oder 2,4-, 3,5- oder 2,6-Dichlorphenyl.

Niederalkyl ist beispielsweise Methyl, Aethyl, Propyl oder n-Butyl oder ferner Isopropyl, sek.-, iso-oder tert.-Butyl.

Niederalkoxy sowie solches in Niederalkoxycarbonyl ist beispielsweise Methoxy, Aethoxy, Propoxy, Isopropoxy, Butoxy oder Amyloxy.

Niederalkanoyl ist beispielsweise Acetyl, Propionyl Butyryl, Isobutyryl, Valeroyl, Pivaloyl oder Caproyl.

Hydroxyniederalkoxy weist bis zu 3, vorzugsweise in höherer als der α-Stellung gebundene, Hydroxygruppen auf und ist insbesondere 2- oder 3-Hydroxyniederalkoxy, beispielsweise 2-Hydroxyäthoxy.

Niederalkylen ist beispielsweise 3- bis 5-, vor alle 3- oder 4-gliedriges Niederalkylen, wie 1,3-Propylen, 1,4-Butylen oder 1,5-Pentylen.

Niederalkylendioxy ist beispielsweise 3- bis 4-gliedriges Niederalkylendioxy, beispielsweise Methylen-

dioxy, Aethylidendioxy, Aethylendioxy oder 1,3-Propylendioxy.

Halogen ist beispielsweise Halogen bis und mit Atomnummer 35, wie Fluor, Chlor oder Brom.

Salze von Verbindungen der allgemeinen Formel (I), worin R für Carboxy steht, sind Salze mit Basen, in erster Linie entsprechende pharmazeutisch verwendbare Salze, wie Alkalimetall- oder Erdalkalimetall-, z.B. Natrium-, Kalium-, Magnesium- oder Calciumsalze, ferner Ammoniumsalze mit Ammoniak oder Aminen, wie Niederalkyl- oder Hydroxyniederalkylaminen, z.B. Trimethylamin, Triäthylamin oder Di-(2-hydroxyäthyl)-amin.

Die neuen Verbindungen zeigen wertvolle pharmakologische Eigenschaften. Insbesondere weisen sie antiallergische Wirkungen auf, wie z.B. an der Ratte in Dosen von etwa 3 bis etwa 100 mg/kg oral bzw. etwa 0,3 bis etwa 10 mg intravenös im passiven kutanen Anaphylaxie-Test (PCA-Reaktion) gezeigt werden kann. Dieser wird analog der von Goose und Blair, Immunology, Bd. 16, S. 749 (1969) beschriebenen Methode durchgeführt, wobei die passive kutane Anaphylaxie nach dem von Ovary, Progr. Allergy, Bd. 5, S. 459 (1958), beschriebenen Verfahren oder durch Hemmung der Histaminfreisetzung, z.B. aus Rattenperitonealzellen in vitro (vgl. Dukor et al., Intern. Arch. Allergy (1976) im Druck) erzeugt wird.

Die neuen Verbindungen sind bekannten antiallergisch wirksamen Mitteln ähnlicher Strucktur, z.B. den in der USA-Patentschrift Nr. 3,937,719 beschriebenen, im 1,2-Phenylenrest durch Alkoxy oder Hydroxyalkoxy mit jeweils bis zu 6 Kohlenstoffatomen substituierten 2-Oxaloamino-4-oxo-4H-1-benzopyranderivaten, wirkungsmässig überlegen.

Die Verbindungen der vorliegenden Erfindung sind dementsprechend vorzüglich verwendbar als Hemmer allergischer Reaktionen, z.B. in der Behandlung und Prophylaxe von

allergischen Erkrankungen, wie Asthma, sowohl extrinsic als auch intrinsic Asthma, oder anderen allergischen Erkrankungen, wie Heufieber, Konjunktivitis, Urticaria und Ekzeme.

Die Erfindung betrifft beispielsweise Verbindungen der Formel I, worin R mit einem gegebenenfalls substituierten Phenylniederalkanol oder einem cycloaliphatischen Alkohol verestertes oder amidiertes Carboxy bedeutet und Ph und $R_1$ die angegebenen Bedeutungen haben, oder worin $R_1$ einen durch gegebenenfalls substituiertes Phenyl substituierten aliphatischen Kohlenwasserstoffrest, einen cycloaliphatischen Kohlenwasserstoffrest oder einen gegebenenfalls substituierten aromatischen Kohlenwasserstoffrest darstellt und R und Ph den angegebenen Bedeutungen haben, oder worin Ph durch Acyl, Hydroxyniederalkoxy oder Trifluoromethyl monosubstituiertes oder durch Niederalkyl, Niederalkoxy, Halogen, Hydroxyniederalkoxy und/oder Hydroxy oder an zwei benachbarten C-Atomen durch Niederalkylen oder Niederalkylendioxy disubstituiertes 1,2-Phenylen darstellt, und R und $R_1$ die angegebenen Bedeutungen haben, jeweils in freier Form oder in Salzform, Verfahren zur Herstellung derselben, diese enthaltende pharmazeutische Präparate und ihre Verwendung als Pharmazeutika oder zur Herstellung pharmazeutischer Präparate.

Die Erfindung betrifft in erster Linie Verbindungen der allgemeinen Formel I, worin R Carboxy, mit einem Alkohol aliphatischen Charakters verestertes Carboxy oder gegebenenfalls durch mindestens einen gegebenenfalls substituierten oder heteroanalogen Kohlenwasserstoffrest aliphatischen Charakters, Hydroxy oder primäres Amino substituiertes Carbamyl bedeutet, Ph gegebenenfalls ein- oder mehrfach durch aliphatische Reste, Acylreste, gegebenenfalls veräthertes oder verestertes Hydroxy und/oder Trifluormethyl substituiertes 1,2-Phenylen bedeutet und $R_1$ Wasserstoff, einen gegebenenfalls substituierten Kohlenwasserstoffrest aliphatischen Charakters oder

aromatischen Kohlenwasserstoffrest bedeutet, wobei als Substituenten von aromatischen Gruppen jeweils vor allem Niederalkyl, Niederalkoxy, Halogen und Trifluormethyl in Betracht kommen, in freier Form oder in Salzform.

Die Erfindung betrifft vor allem Verbindungen der allgemeinen Formel I, worin R Carboxy, mit einem Niederalkanol verestertes Carboxy oder gegebenenfalls durch Niederalkyl monosubstituiertes oder in zweiter Linie durch Niederalkyl, Niederalkylen oder 3-Oxa-, 3-Aza- oder 3-Thia-niederalkylen disubstituiertes Carbamyl bedeutet, Ph gegebenenfalls durch Niederalkyl, wie Methyl, 3- oder 4-gliedriges Niederalkylen, wie 1,3-Propylen, Hydroxy, Niederalkoxy, wie Methoxy, Hydroxyniederalkoxy, worin die Hydroxygruppe in höherer als der α-Stellung gebunden ist, wie 2-Hydroxyäthoxy, 3- oder 4-gliedriges Niederalkylendioxy, wie Methylendioxy oder Aethylendioxy, Niederalkanoyl, wie Acetyl oder Butyryl, Trifluormethyl und/oder Halogen, wie Chlor, substituiertes 1,2-Phenylen bedeutet, und $R_1$ Wasserstoff oder in zweiter Linie Niederalkyl darstellt, in freier Form oder in Salzform.

Die Erfindung betrifft insbesondere Verbindungen der allgemeinen Formel I, worin R Carboxy oder in zweiter Linie Niederalkoxycarbonyl, wie Methoxy- oder Aethoxycarbonyl, oder gegebenenfalls durch Niederalkyl, wie Methyl oder Aethyl, mono- oder disubstituiertes Carbamyl bedeutet, Ph gegebenenfalls durch Niederalkyl, wie Methyl, 3- oder 4-gliedriges Niederalkylen, wie 1,3-Propylen, Hydroxy, Niederalkoxy, wie Methoxy, 2- oder 3-Hydroxy-niederalkoxy, wie 2-Hydroxyäthoxy, 3- oder 4-gliedriges Niederalkylendioxy, wie Methylendioxy oder Aethylendioxy, Niederalkanoyl, wie Acetyl oder Butyryl, und/oder Halogen, wie Chlor substituiertes 1,2-Phenylen bedeutet und $R_1$ Wasserstoff oder in zweiter Linie Niederalkyl, wie Methyl, bedeutet, in freier Form oder in Salzform.

Die Erfindung betrifft in allererster Linie Verbindungen der allgemeinen Formel I, worin R Carboxy oder in zweiter Linie Niederalkoxycarbonyl mit bis zu 5 C-Atomen, wie Methoxy- oder Aethoxycarbonyl bedeutet, Ph in einer der freien Stellungen gegebenenfalls durch Niederalkyl mit bis zu 4 C-Atomen, wie Methyl, 3- oder 4-gliedriges Niederalkylen mit bis zu 4 C-Atomen, wie 1,3-Propylen, Niederalkoxy mit bis zu 4 C-Atom, wie Methoxy, 3- oder 4-gliedriges Niederalkylendioxy, wie Methylendioxy oder Aethylendioxy, Niederalkanoyl mit bis zu 7 C-Atomen, wie Acetyl oder Butyryl, Hydroxy und/oder Halogen bis Atomnummer 35, wie Chlor, substituiertes 1,2-Phenylen bedeutet, und $R_1$ Wasserstoff, Niederalkyl mit bis zu 4 C-Atomen, wie Methyl, oder Phenyl ist, jeweils in freier Form oder in Salzform.

Die Erfindung betrifft in allererster Linie beispielsweise Verbindungen der allgemeinen Formel I, worin R Carboxy bedeutet, Ph unsubstituiertes, durch Niederalkyl mit bis zu 4 C-Atomen, wie Methyl, Niederalkoxy mit bis zu 4 C-Atomen, wie Methoxy, Niederalkanoyl mit bis zu 7 C-Atomen, wie Butyryl, oder Halogen bis Atomnummer 35, wie Chlor, monosubstituiertes oder durch Niederalkyl mit bis zu 4 C-Atomen, wie Methyl, Niederalkyloxy mit bis zu 4 C-Atomen, wie Methoxy, Halogen bis Atomnummer 35, wie Chlor, und/oder Niederalkanoyl mit bis zu 7 C-Atomen, wie Butyryl, oder an zwei benachbarten C-Atomen durch 3- oder 4-gliedriges Niederalkylen mit bis zu 4 C-Atomen, wie 1,3-Propylen, disubstituiertes 1,2-Phenylen bedeutet und $R_1$ Wasserstoff darstellt, in freier Form oder in Salzform.

Die Erfindung betrifft ganz besonders Verbindungen der Formel Ia

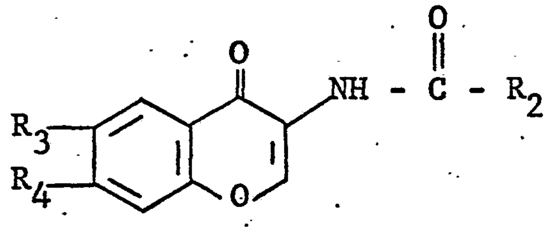

(Ia) ,

worin $R_2$ Carboxy bedeutet und $R_3$ und $R_4$ unabhängig voneinander Wasserstoff, Niederalkyl mit bis zu 4 C-Atomen, wie Methyl, Niederalkoxy mit bis zu 4 C-Atomen, wie Methoxy, Niederalkanoyl mit bis zu 7, z.B. bis zu 4 C-Atomen, wie Acetyl oder Butyryl, Hydroxy oder Halogen bis Atomnummer 35, wie Chlor, bedeuten oder gemeinsam einen Niederalkylen- oder Niederalkylendioxyrest mit bis zu 4 C-Atomen, wie 1,3-Propylen, Methylendioxy oder Aethylendioxy, darstellen, in freier Form oder in Salzform.

Die Erfindung betrifft vorzugsweise Verbindungen der Formel Ia, worin $R_2$ Carboxy bedeutet und $R_3$ und $R_4$ entweder Wasserstoff oder unabhängig voneinander $C_1$-$C_4$-Alkyl, wie Methyl, oder gemeinsam $C_3$- oder $C_4$-Alkylen, wie Propylen-1,3 darstellen, in freier Form oder in Salzform.

Die Erfindung betrifft namentlich die in den Beispielen genannten Verbindungen der Formel I in freier Form oder in Salzform.

Die neuen Verbindungen können nach an sich bekannten Verfahren hergestellt werden.

Eine bevorzugte Arbeitsweise ist beispielsweise dadurch gekennzeichnet, dass man eine Verbindung der allgemeinen Formel II

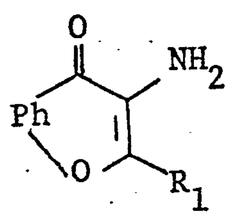

(II)

oder ein Säureadditionssalz davon mit einer Verbindung der Formel R-X (III) umsetzt, worin X eine gegebenenfalls funktionell abgewandelte Carboxygruppe bedeutet, und gewünschtenfalls eine so erhältliche Verbindung in eine andere Verbindung der Formel I umwandelt und/oder ein erhaltenes Salz in die freie Verbindung oder in ein anderes Salz oder eine erhaltene salzbildende Verbindung in ein Salz überführt.

Säureadditionssalze von Verbindungen der Formel II sind beispielsweise Hydrohalogenide, wie Hydrochloride, derselben, ferner Salze mit Säuren der Formel III.

Funktionell abgewandelte Carboxygruppen X sind beispielsweise veresterte, amidierte oder anhydridisierte Carboxygruppen, wie Niederalkoxycarbonyl, gegebenenfalls substituiertes Carbamyl, z.B. Carbamyl, Diniederalkylcarbamyl oder Imidazolyl-1-carbonyl, oder Halogencarbonyl, z.B. Chlor- oder Bromcarbonyl. Als Beispiele für Ausgangsstoffe der Formel III seien insbesondere genannt: Oxalsäure, Oxalsäurediester, wie Oxalsäurediniederalkylester und gegebenenfalls veresterte oder amidierte Halogenoxalsäuren, wie Chlor- oder Bromoxalsäureniederalkylester oder -diniederalkylamide, namentlich Oxalsäurediäthylester und Brom- bzw. Chloroxalsäureäthylester.

Die Umsetzung von Verbindungen der Formeln II und III kann in üblicher Weise erfolgen, beispielsweise in Gegenwart eines wasserbindenden Mittels, wie eines Säureanhydrides, z.B. von Phosphorpentoxid, oder von Dicyclohexylcarbodiimid, oder eines, z.B. sauren oder basischen, Kondensationsmittels, wie einer Mineralsäure, z.B. von Chlorwasserstoffsäure, oder eines Alkalimetallhydroxydes oder -carbonates, z.B. von Natrium- oder Kaliumhydroxyd, oder einer organischen Stickstoffbase, z.B. von Triäthylamin oder Pyridin. Bei der Umsetzung mit einem Esterhalogenid oder Amidhalogenid der Oxalsäure verwendet man vorzugsweise eine organische Stickstoffbase als Kondensationsmittel. Die Umsetzung mit Oxalsäure führt man vorzugsweise in Gegenwart eines die Dehydratisierung des primär gebildeten substituierten Ammoniumsalzes bewirkenden wasserbindenden Mittels oder sauren Kondensationsmittels aus. Erforderlichenfalls arbeitet man jeweils in einem inerten Lösungs- oder Verdünnungsmittel, wie einem Kohlenwasserstoff, z.B. Toluol, N,N-Diniederalkylamid, z.B. Dimethylformamid, oder in Chloroform oder Methylenchlorid, bei normaler Temperatur oder unter Kühlen oder Erwärmen, z.B. im Temperaturenbereich von etwa 0° bis 100°C, in einem geschlossenen Gefäss und/oder unter Inertgas, z.B. unter Stickstoff.

Die Ausgangsstoffe der Formel II sind bekannt oder können nach an sich bekannten Methoden hergestellt werden, beispielsweise indem man in einem entsprechenden 3-Nitro-4-oxo-4H-1-benzopyranderivat die Nitrogruppe in üblicher Weise, z.B. durch Behandeln mit durch Palladium auf Aktivkohle katalytisch aktiviertem Wasserstoff, z.B. in Dimethylformamid bei Normaldruck, zur Aminogruppe reduziert.

Die vorstehend erwähnten 3-Nitro-4-oxo-4H-1-benzopyranderivate sind ihrerseits bekannt oder können nach an sich bekannten Methoden hergestellt werden, beispielsweise indem man ein entsprechendes Methylsulfinylacetophenon der Formel HO-Ph-C(=O)-$CH_2$-S(=O)-$CH_3$ in Gegenwart einer Base, z.B. von Kaliumcarbonat in Wasser, mit einem Aldehyd der Formel $R_1$-CHO, z.B. Formaldehyd, kondensiert, aus dem so erhältlichen 2-$R_1$-3-Hydroxymethyl-3-methylsulfinyl-2,3-dihydro-4-oxo-4H-1-benzopyranderivat thermisch, z.B. in siedendem Toluol, Sulfinsäure abspaltet und das so erhältliche 2-$R_1$-3-hydroxymethyl-4-oxo-4H-benzopyranderivat mit konzentrierter, z.B. 70%-iger Salpetersäure, mässig, z.B. auf etwa 40°C, erwärmt. Eine direkte Bildungsweise für die erwähnten Nitroverbindungen besteht darin, dass man ein entsprechendes α-Nitroacetophenon der Formel HO-Ph-C(=O)-$CHR_1$-$NO_2$ in Gegenwart von Natriumformiat mit dem gemischten Anhydrid von Essig- und Ameisensäure, z.B. auf Siedetemperatur erhitzt.

Die neuen Verbindungen können ferner hergestellt werden, indem man in einer Verbindung der Formel

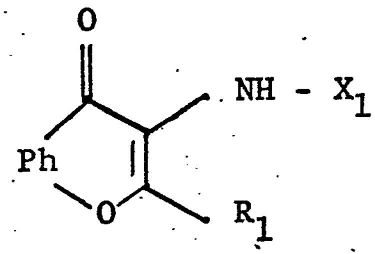

(IV),

worin $X_1$ einen in die gewünschte Gruppe der Formel R-C(=O)-überführbaren Rest bedeutet, $X_1$ in die Gruppe der Formel R-C(=O)- überführt und gewünschtenfalls eine so erhältliche

Verbindung in eine andere Verbindung der Formel I umwandelt und/oder ein erhaltenes Salz in die freie Verbindung oder in ein anderes Salz oder eine erhaltene salzbildende Verbindung in ein Salz überführt.

Ein in die Gruppe der Formel R-C(=O)- überführbarer Rest ist beispielsweise eine von einer gegebenenfalls veresterten oder amidierten Oxalogruppe verschiedene und in diese überführbare funktionell abgewandelte Oxalogruppe. Derartige funktionell abgewandelte Oxalogruppen sind vorzugsweise solche, die als funktionell abgewandelte α-Carbonylgruppierung Thioxomethylen, Iminomethylen oder eine veresterte und/oder verätherte Dihydroxymethylengruppierung und/oder als funktionell abgewandelte Carboxygruppe eine von einer veresterten oder amidierten Carboxygruppe verschiedene funktionell abgewandelte Carboxygruppe aufweisen. Veresterte und/oder verätherte Dihydroxymethylgruppierungen sind beispielsweise mit einer Halogenwasserstoffsäure, wie Chlorwasserstoffsäure, veresterte und/oder mit einem Niederalkanol, wie Methanol oder Aethanol, verätherte Dihydroxymethylengruppen. Als Beispiele seien vor allem Dihalogenmethylengruppierungen, wie Dichlormethylen, Niederalkoxyhalogenmethylengruppierungen, wie Methoxy- oder Aethoxychlormethylen, oder Diniederalkoxymethylengruppierungen, wie Dimethoxy- oder Diäthoxymethylen, genannt. Von veresterten oder amidierten Carboxylgruppen verschiedene funktionell abgewandelte Carboxygruppierungen sind beispielsweise die Cyanogruppe, anhydridisierte Carboxygruppen, wie Halogen-, z.B. Chlorcarbonyl, Iminoester-, wie Imid- bzw. Amidhalogenidgruppierungen, z.B. Iminochlor- oder Aminodichlormethyl, Iminoäthergruppierungen, wie Niederalkyl- oder Niederalkyleniminoäthergruppierungen, z.B. Methoxy- oder Aethoxyiminomethyl, 4,4- oder 5,5-Dimethyloxazolinyl-(2) oder 4,4,6-Trimethyl-dihydro-oxazinyl-(2), Amidinogruppen, wie Amidino oder Niederalkyl-, z.B. Methylamidino, mit einer Halogenwasserstoffsäure, wie Chlorwasserstoffsäure, veresterte und/oder mit einem Niederalkanol verätherte Orthosäuregruppierungen, wie

Triniederalkoxy-, Niederalkoxydihalogen- oder Trihalogenmethylgruppen, vor allem Trimethoxy- oder Triäthoxymethyl, Aethoxydichlormethyl oder Trichlormethyl, oder gegebenenfalls veresterte Thiocarboxylgruppen, wie Niederalkylthiocarbonylgruppen, z.B. Aethylthiocarbonyl.

Derartige Gruppen $X_1$ können solvolytisch in die Gruppe der Formel R-C(=O)-, z.B. hydrolytisch in die Oxalogruppe, überführt werden. Als funktionell abgewandelte Carboxygruppe eine Iminoäther-, Orthoester- oder Esterhalogenidgruppierung und/oder als funktionell abgewandelte α-Carbonylgruppe Thioxo- oder Iminomethylen oder eine veresterte oder verätherte Dihydroxymethylengruppe aufweisende Gruppen $X_1$ können ferner zu veresterten Oxalogruppen hydrolysiert werden. Ebenso können als funktionell abgewandelte Carboxygruppe die Cyanogruppe, eine Amidino- oder Imid- bzw. Amidhalogenidgruppierung und/oder als funktionell abgewandelte α-Carbonylgruppe Thioxo- oder Iminomethylen oder eine verätherte oder veresterte Dihydroxymethylengruppe aufweisende Gruppen $X_1$ zu amidierten Carboxygruppen hydrolysiert werden. Die Hydrolyse kann in üblicher Weise durchgeführt werden, erforderlichenfalls in Gegenwart eines basischen oder vorzugsweise sauren Hydrolysemittels, wie eines Alkalimetallhydroxydes, wie Natrium- oder Kaliumhydroxyd, oder vorzugsweise einer Protonensäure, vor allem einer Mineralsäure, z.B. einer Halogenwasserstoffsäure, wie Salzsäure, oder einer organischen Carbon- oder Sulfonsäure, z.B. von Essigsäure oder p-Toluolsulfonsäure, erforderlichenfalls in einem polaren Lösungsmittel, wie einem Niederalkanol, Keton oder Aether, z.B. in Aethanol, Aceton oder Dioxan, und/oder unter Kühlen oder Erwärmen, z.B. bei etwa 0°C bis etwa 100°C.

Als funktionell abgewandelte Carboxygruppe eine anhydridisierte Carboxygruppe, wie Halogencarbonyl, z.B. Chlorcarbonyl, oder eine Niederalkyleniminoäthergruppierung, z.B. 4,4- oder 5,5-Dimethyl-oxazolinyl-(2) oder 4,4,6-Trimethyl-dihydro-oxazinyl-(2), aufweisende funktionell abgewandelte

Oxalogruppen können ferner durch übliche Alkoholyse, d.h. Umsetzung mit dem entsprechenden Alkohol, in veresterte Oxalogruppen überführt werden. Bei der Alkoholyse von anhydridisierten Carboxygruppen arbeitet man vorteilhaft in Gegenwart eines basischen Kondensationsmittels, z.B. von Pyridin oder Triäthylamin, während man die Alkoholyse einer Niederalkyleniminoäthergruppierung vorzugsweise sauer, z.B. in Gegenwart von Chlorwasserstoffsäure, p-Toluolsulfonsäure oder Essigsäure durchführt. In analoger Weise kann man eine eine anhydridisierte Carboxygruppe aufweisende funktionell abgewandelte Oxalogruppe auch durch Ammono- bzw. Aminolyse, d.h. Umsetzung mit Ammoniak oder eines entsprechenden primären oder sekundären Amin, vorzugsweise in Gegenwart eines basischen Kondensationsmittels, z.B.von Natriumhydroxid, Pyridin oder Triäthylamin, in eine amidierte Oxalogruppe R-C(=O)- überführen.

Weitere in Gruppen der Formel R-C(=O)- überführbare Reste $X_1$ sind beispielsweise oxydativ in diese überführbare Gruppen, insbesondere die oxydativ in die Oxalogruppe der Formel R-C(=O)-, worin R Carboxy darstellt, überführbare, gegebenenfalls hydratisierte Glyoxyloylgruppe. Diese kann vorteilhaft im Verlaufe der Oxydationsreaktion, z.B. aus der Acylgruppe einer gegebenenfalls α,β-ungesättigten oder α,β-dihydroxylierten aliphatischen oder araliphatischen Carbonsäure, einer gegebenenfalls an der Hydroxygruppe veresterten Glykoloylgruppe oder der Glycylgruppe, <u>in situ</u> gebildet oder aus einem ihrer funktionellen Derivate, z.B. einem ihrer Acetale oder Imine in Freiheit gesetzt werden. Acylgruppen von gegebenenfalls α,β-ungesättigten oder α,β-dihydroxylierten Carbonsäuren sind beispielsweise Alkanoylgruppen, wie Niederalkanoyl, z.B. Acetyl, Acylgruppen von α,β-ungesättigten aliphatischen Mono- oder Dicarbonsäuren, z.B. Acryloyl, Crotonyl oder die Acylgruppe der gegebenenfalls funktionell abgewandelten Fumar- oder Maleinsäure, Acylgruppen von α,β-ungesättigten araliphatischen Carbonsäuren, z.B. gegebenenfalls substituiertes Cinnamoyl, oder Acylgruppen von aliphatischen $\alpha,\beta$-Dihydroxydicarbonsäuren, wie der Weinsäure, oder monofunktioneller Carboxyderivate, wie Estern oder Amiden, derselben. Veresterte Glykoloylgruppen sind beispielsweise an der Hydroxygruppe mit einer Mineralsäure, wie einer Halogenwasserstoffsäure, z.B. mit Chlor- oder Bromwasserstoffsäure, oder mit einer Carbonsäure, z.B. mit Essigsäure oder der gegebenenfalls substituierten Benzoesäure, veresterte Glykoloylgruppen. Acetalisierte Glyoxyloylgruppen sind beispielsweise mit Niederalkanolen oder einem Niederalkandiol acetalisierte Glyoxyloylgruppen, wie Dimethoxy-, Diäthoxy- oder Aethylendioxyacetyl. Imine von Glyoxyloylgruppen sind beispielsweise gegebenenfalls substituierte N-Benzylimine derselben. Weitere oxydativ in die Oxalogruppe überführbare Reste sind z.B. gegebenenfalls substituierte, wie in 5-Stellung eine acetalisierte Formylgruppe, wie Diäthoxymethyl, aufweisende 2-Furoylgruppen. Zu veresterten Oxalogruppen der Formel R-C(=O)-, worin R für verestertes Carboxy steht, oxydierbare Gruppen sind verätherte Glykoloylgruppen, wie Niederalkoxyacetyl. Zu gegebenenfalls veresterten oder amidierten Oxalogruppen oxydierbaren Reste sind ferner gegebenenfalls veresterte oder amidierte Carboxymethylgruppen.

Die Oxydation derartiger Gruppen $X_1$ kann in üblicher Weise durch Umsetzung mit einem geeigneten Oxydationsmittel erfolgen. Geeignete Oxydationsmittel sind insbesondere oxydierende Schwermetallverbindungen, wie Silberverbindungen, z.B. Silbernitrat oder Silberpicolinat, Sauerstoffsäuren von Schwermetallen, z.B. von Mangan-IV und -VII, Blei-IV, Chrom-VI oder Eisen-VI, oder von Halogenen bzw. deren Anhydride oder Salze, wie Chromsäure, Chromtrioxid, Kaliumdichromat, Kaliumpermanganat, Mangandioxid, Kaliumferrat, Natriumjodat, Natriumperjodat oder Bleitetraacetat. Die Umsetzung mit diesen Oxydationsmitteln erfolgt in üblicher Weise, beispielsweise in einem inerten Lösungsmittel, wie Aceton, Schwefelsäure, Pyridin, oder Wasser, oder einem, vorzugsweise wässrigen, inerten Lösungsmittelgemisch, bei Normaltemperatur oder erforderlichenfalls unter Kühlen oder Erwärmen, z.B. bei etwa 0°C bis etwa 100°C. Die Oxydation von gegebenenfalls verätherten Glykoloylgruppen zu gegebenenfalls veresterten Oxalogruppen wird z.B. vorteilhaft mit Kaliumpermanganat in wässrigem Pyridin oder Aceton bei Raumtemperatur vorgenommen. Acetalisierte Glyoxyloylgruppen und Iminoacetylgruppen werden vorzugsweise sauer oxydiert, z.B. mit Kaliumdichromat in Schwefelsäure. Acylgruppen von α,β-dihydroxylierten aliphatischen Carbonsäuren, wie der Acylrest der Weinsäure werden vorteilhaft mit Perjodsäure oxydiert, während man für die Oxydation der Glycylgruppe vorzugsweise Kaliumferrat in alkalischem Milieu, z.B. bei $p_H$ = 10-13, z.B. 11.5, oder organische Silbersalze, wie Silberpicolinat verwendet.

Die als Ausgangsstoffe genannten Verbindungen der Formel IV sind grösstenteils neu. Sie weisen teilweise neben ihrer Verwendbarkeit als Ausgangsstoffe für die Herstellung von Verbindungen der Formel I weitere vorteilhafte Eigenschaften auf. So besitzen Verbindungen der Formel IV, in denen $X_1$ eine gegebenenfalls veresterte oder verätherte Glykoloylgruppe bedeutet, die gleichen pharmakologischen Eigenschaften, in vergleichbarer Wirkungsstärke, wie die entsprechenden Verbindungen der Formel I.

Die Erfindung betrifft dementsprechend ebenfalls neue Ausgangsstoffe, vor allem Verbindungen der Formel IV, worin $X_1$ eine gegebenenfalls veresterte oder verätherte Glykoloylgruppe bedeutet, Verfahren zu deren Herstellung, diese enthaltende pharmazeutische Präparate und ihre Verwendung als Pharmazeutika oder zur Herstellung von Arzneimitteln.

Dabei sind unter veresterten Glykoloylgruppen beispielsweise mit einer Carbonsäure, wie einer aliphatischen oder aromatischen Carbonsäure, veresterte Glykoloylgruppen zu verstehen, z.B. entsprechendes Niederalkanoyloxyacetyl oder gegebenenfalls substituiertes Benzoyloxyacetyl. Niederalkanoyloxyacetyl ist z.B. Acetoxy-, Propionyloxy-, Butyryloxy-, Isobutyryloxy-, Valeroyloxy-, Caproyloxy- oder Pivaloyloxyacetyl. Als Substituenten von substituierten Benzoyloxyacetylgruppen kommen vor allem Niederalkyl, wie Methyl, Niederalkoxy, wie Methoxy und/oder Halogen, wie Chlor, in Betracht.

Verätherte Glykoloylgruppen sind beispielsweise durch einen gegebenenfalls substituierten aliphatischen oder araliphatischen Alkohol verätherte Glykoloylgruppen, wie entsprechende Niederalkoxyacetyl- oder Phenylniederalkoxyacetylgruppen. Substituenten von Niederalkoxyacetyl sind vor allem Hydroxy, Niederalkoxy und/oder Diniederalkylamino, und solche von Phenylniederalkoxyacetylgruppen z.B. Niederalkyl, wie Methyl, Niederalkoxy, wie Methoxy, und/oder Halogen, wie Chlor. Niederalkoxy hat dabei vorzugsweise eine der eingangs angeführten Bedeutungen. Phenylniederalkoxyacetyl ist insbesondere Benzyloxy- oder 2-Phenyläthoxyacetyl. Diniederalkylaminoniederalkoxyacetyl ist vorzugsweise 2-Dimethyl- oder 2-Diäthylaminoäthoxyacetyl.

Die Erfindung betrifft dabei insbesondere solche Verbindungen der Formel IV, in denen Ph und $R_1$ die für die jeweils bevorzugten Verbindungsgruppen der Formel I angegebenen Bedeutungen haben, und $X_1$ Niederalkoxyacetyl, vor allem mit bis zu 6 Kohlenstoffatomen, wie Methoxy- oder Aethoxyacetyl, oder vorzugsweise Glykoloyl bedeutet.

Die als Ausgangsstoffe genannten Verbindungen der Formel IV können nach an sich bekannten Methoden hergestellt werden, vorzugsweise, indem man eine Verbindung der Formel

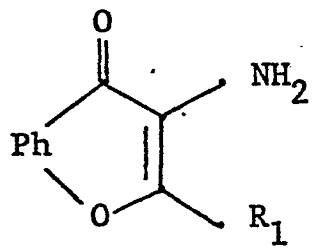

(II)

oder ein Säureadditionssalz davon mit einer Säure der Formel $X_1$-OH (IVa) oder einem funktionellen Derivat davon umsetzt.

Funktionelle Derivate von Säuren der Formel IVa sind beispielsweise eine veresterte, amidierte oder anhydridisierte Carboxygruppe, wie Niederalkoxycarbonyl, gegebenenfalls substituiertes Carbamyl, z.B. Carbamyl, Diniederalkylcarbamyl oder Imidazolyl-1-carbonyl, oder Halogencarbonyl, z.B. Chlor- oder Bromcarbonyl, enthaltende Säurederivate. Als Beispiele für Säuren der Formel IVa und deren funktionelle Derivate seien insbesondere genannt: für die Herstellung von Verbindungen der Formel IV, in denen $X_1$ einen zur Gruppe R-C(=O)- solvolysierbaren Rest bedeutet, Oxalylhalogenide, wie Oxalylchlorid oder Oxalylbromid, Triniederalkoxy- und Dihalogen-niederalkoxy-essigsäureniederalkylester, wie Oxalsäuretetraäthylester oder Dichloroxalsäurediäthylester, Oxalsäureiminodialkylester, wie Oxalsäuremono- oder -diiminodiäthylester, Oxalsäureamidine, wie N-Niederalkyloxalsäureesteramidine, Oxalsäuredithioniederalkyl-, wie -dimethylester, Cyanoformylchlorid und Cyanogen, und für die Herstellung von Verbindungen der Formel IV, in denen $X_1$ einen zur Gruppe R-C-(=O)- oxydierbaren Rest bedeutet, Glykolsäure und ihre Niederalkylester bzw. das entsprechende Lactid, Mono- oder Diniederalkoxyessigsäure und deren Niederalkylester, z.B. Aethoxy- oder Diäthoxyessigsäureäthylester, Halogenacetanhydride, wie Chloracetanhydrid oder Chloracetylchlorid und Weinsäure, ferner Cinnamoylchlorid, Acetylchlorid und Glycin.

Die Umsetzung von Verbindungen der Formel II mit

Säuren der Formel IVa bzw. deren Derivaten kann in üblicher Weise erfolgen, beispielsweise in Gegenwart eines wasserbindenden Mittels, wie eines Säureanhydrides, z.B. von Phosphorpentoxid, oder von Dicyclohexylcarbodiimid, oder eines, z.B. sauren oder basischen, Kondensationsmittels, wie einer Mineralsäure, z.B. von Chlorwasserstoffsäure, oder eines Alkalimetallhydroxydes oder -carbonates, z.B. von Natrium- oder Kaliumhydroxyd, oder einer organischen Stickstoffbase, z.B. von Triäthylamin oder Pyridin. Bei der Umsetzung mit einem Säureanhydrid, wie Säurechlorid, verwendet man vorzugsweise eine organische Stickstoffbase als Kondensationsmittel. Die Umsetzung mit Carbonsäuren führt man vorzugsweise in Gegenwart eines wasserbindenden Mittels durch. Erforderlichenfalls arbeitet man jeweils in einem inerten Lösungsmittel, bei normaler Temperatur oder unter Kühlen oder Erwärmen, z.B. im Temperaturbereich von etwa 0°C bis etwa 100°C, in einem geschlossenen Gefäss und/oder unter Inertgas, z.B. Stickstoff.

Verbindungen der Formel IV, in denen $X_1$ für Glyoxyloyl steht, können ferner hergestellt werden, indem man eine entsprechende Halogen-, wie Bromacetylverbindung mit Hexamethylentetramin, vorzugsweise in einem wässrigen Alkohol, erhitzt. Verbindungen der Formel IV, in denen $X_1$ gegebenenfalls substituiertes Benzyliminoacetyl bedeutet, können ausgehend von den entsprechenden Glycylverbindungen hergestellt werden, indem man diese mit gegebenenfalls substituiertem Benzaldehyd umsetzt und die so erhältliche Benzylidenglycylverbindung, vorzugsweise unter den Reaktionsbedingungen, umlagert.

Als funktionell abgewandelte Carboxygruppe eine Iminoäthergruppierung aufweisende funktionell abgewandelte Oxalogruppen aufweisende Verbindungen der Formel IV können ausgehend von der entsprechenden Cyanocarbonylverbindung durch Umsetz Umsetzung mit dem entsprechenden Alkohol, cyclische Iminoäther durch Behandeln mit einem Niederalkandiol oder Aminoniederalkanol, hergestellt werden.

Die erfindungsgemässen Verbindungen der Formel IV, in denen $X_1$ eine gegebenenfalls verätherte oder mit einer Carbonsäure veresterte Glykoloylgruppe bedeutet, können ferner hergestellt werden, indem man in einer Verbindung der Formel

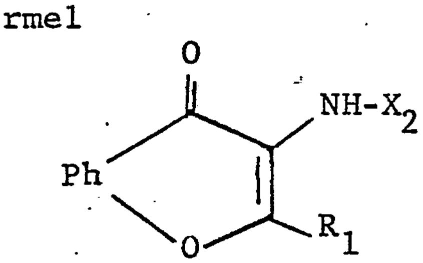

(IV'),

worin $X_2$ einen in die genannte Gruppe $X_1$ überführbaren Rest bedeutet, den Rest $X_2$ in einen Rest $X_1$ überführt und gewünschtenfalls eine so erhältliche Verbindung in eine andere Verbindung der Formel IV, worin $X_1$ eine gegebenenfalls mit einer Carbonsäure veresterte oder verätherte Glykoloylgruppe bedeutet, überführt.

Derartige Gruppen $X_2$ sind beispielsweise von der gegebenenfalls mit einer Carbonsäure veresterten Glykoloylgruppe verschiedene veresterte Glykoloylgruppen, wie mit einer Mineralsäure, z.B. mit einer Halogenwasserstoffsäure, veresterte Glykoloylgruppen, wie Chlor-, Brom- oder Jodacetyl. Diese Gruppen können hydrolytisch, z.B. in Gegenwart eines basischen Hydrolysemittels, wie Natronlauge zur Glykoloylgruppe oder durch Umsetzung mit einem Salz, z.B. dem Natriumsalz, eines entsprechenden Alkohols bzw. einer entsprechenden Carbonsäure in veräthertes bzw. mit einer Carbonsäure verestertes Glykoloyl überführt werden.

Weitere in Gruppen $X_1$ überführbare Reste $X_2$

sind z.B. reduktiv in die Glykoloylgruppe überführbare Reste, wie die gegebenenfalls hydratisierte Glyoxylgruppe, die auch unten den Reaktionsbedingungen aus der gegebenenfalls in Salz- oder Anhydrid- bzw. Esterform vorliegenden Oxalogruppe gebildet oder aus einem Acetal, wie Diäthyl- oder Aethylenacetal, hydrolytisch in Freiheit gesetzt werden kann. Als Reduktionsmittel verwendet man z.B. Leicht- oder Dileichtmetallhydride, wie Boran, Diboran, Natriumboranat oder Lithiumanilinoborhydrid.

Eine erfindungsgemäss erhältliche Verbindung der Formel I bzw. IV kann in an sich bekannter Weise in eine andere Verbindung der Formel I bzw. IV umgewandelt werden.

So kann man beispielsweise eine freie Carboxylgruppe R in üblicher Weise, z.B. durch Behandeln mit einem gegebenenfalls durch gegebenenfalls substituiertes Phenyl substituierten Diazoniederalkan oder einen Triniederalkyloxonium-, Triniederalkylcarboxonium- oder Diniederalkylcarboniumsalz, wie -hexachloroantimonat oder -hexafluorophosphat, oder vor allem durch Umsetzung mit dem entsprechenden Alkohol oder einem reaktionsfähigen Ester, wie einem Carbon-, Phosphorig-, Schweflig- oder Kohlensäureester, z.B. einem Niederalkancarbonsäureester, Triniederalkylphosphit, Diniederalkylsulfit oder dessen Carbonat oder Pyrocarbonat, oder einem Mineralsäure- oder Sulfonsäureester, z.B. dem Chlor- oder Bromwasserstoffsäure- oder Schwefelsäure-, Benzolsulfonsäure-, Toluolsulfonsäure- oder Methansulfonsäureester, des entsprechenden Alkohols oder einem davon abgeleiteten Olefin zu einer veresterten Carboxylgruppe R verestern.

Die Umsetzung mit dem entsprechenden Alkohol selbst kann vorteilhaft in Gegenwart eines sauren Katalysators erfolgen, wie einer Protonensäure, z.B. von Chlor- oder Bromwasserstoff-, Schwefel-, Phosphor-, Bor-, Benzolsulfon- und/oder Toluolsulfonsäure, oder einer Lewissäure, z.B. von Bortrifluorid Aetherat, in einem inerten Lösungsmittel, insbesondere einem Ueberschuss des eingesetzten Alkohols und erforderlichenfalls in Gegenwart eines wasserbindenden Mittels und/oder unter

destillativer, z.B. azeotroper, Entfernung des Reaktionswassers und/oder bei erhöhter Temperatur.

Die Umsetzung mit einem reaktionsfähigen Derivat des entsprechenden Alkohols kann in üblicher Weise durchgeführt werden, bei der Umsetzung mit einem Carbon-, Phosphorig-, Schweflig- oder Kohlensäureester beispielsweise in Gegenwart eines sauren Katalysators, wie eines der vorstehend genannten, in einem inerten Lösungsmittel, wie einem aromatischen Kohlenwasserstoff, z.B. in Benzol oder Toluol, oder einem Ueberschuss des eingesetzten Alkoholderivates oder des entsprechenden Alkohols, erforderlichenfalls unter, z.B. azeotroper, Abdestillation des Reaktionswassers. Bei der Umsetzung mit einem Mineralsäure- oder Sulfonsäureester setzt man die zu veresternde Säure vorteilhaft in Form eines Salzes z.B. des Natrium- oder Kaliumsalzes, ein und arbeitet erforderlichenfalls in Gegenwart eines basischen Kondensationsmittels, wie einer anorganischen Base, z.B. von Natrium- oder Kalium- oder Calciumhydroxid oder -carbonat, oder einer tertiären organischen Stickstoffbase, z.B. von Triäthylamin oder Pyridin, und/oder in einem inerten Lösungsmittel, wie einer der vorstehenden tertiären Stickstoffbasen oder einem polaren Lösungsmittel, z.B. in Dimethylformamid,und/oder bei erhöhter Temperatur.

Die Umsetzung mit einem Olefin kann beispielsweise in Gegenwart eines sauren Katalysators, z.B. einer Lewissäure, z.B. von Bortrifluorid, einer Sulfonsäure, z.B. von p-Toluolsulfonsäure, oder eines basischen Katalysators, z.B. von Natrium- oder Kaliumhydroxid, vorteilhaft in einem inerten Lösungsmittel, wie einem Aether, z.B. in Diäthyläther oder Tetrahydrofuran, erfolgen.

Eine freie Carboxylgruppe R kann ferner durch Umsetzung mit Ammoniak oder einem mindestens ein Wasserstoffatom

aufweisenden Amin in üblicher Weise unter Dehydratisierung des intermediär gebildeten Ammoniumsalzes, z.B. durch azeotrope Destillation mit Benzol oder Toluol oder trockenes Erhitzen, in eine amidierte Carboxylgruppe R überführt werden.

Die vorstehend beschriebenen Umwandlungen freier in veresterte oder amidierte Carboxylgruppen R können aber auch so durchgeführt werden, dass man eine Verbindung der Formel (I), worin R Carboxyl ist, zunächst in üblicher Weise in ein reaktionsfähiges Derivat, beispielsweise mittels eines Halogenides des Phosphors oder Schwefels, z.B. mittels Phosphortrichlorid oder -bromid, Phosphorpentachlorid oder Thionylchlorid, in ein Säurehalogenid oder durch Umsetzung mit einem entsprechenden Alkohol in einen reaktiven Ester, d.h. Ester mit elektronenanziehenden Strukturen, wie den Ester mit Phenol, Thiophenol, p-Nitrophenol oder Cyanmethylalkohol, oder mit einem entsprechenden Amin in ein reaktives Amid, z.B. das von Imidazol oder 3,5-Dimethylpyrazol abgeleitete Amid, überführt. Das erhaltene reaktionsfähige Derivat kann dann in üblicher Weise, z.B. wie nachstehend für die Umesterung, Umamidierung bzw. gegenseitige Umwandlung veresterter und amidierter Carboxylgruppen R beschrieben, mit einem entsprechenden Alkohol, Ammoniak oder dem entsprechenden, mindestens ein Wasserstoffatom aufweisenden Amin zu der gewünschten Verbindung der Formel I umgesetzt werden.

Eine veresterte Carboxylgruppe R kann in üblicher Weise, z.B. durch Hydrolyse in Gegenwart eines Katalysators, beispielsweise eines basischen oder sauren Mittels, wie einer starken Base, z.B. von Natrium- oder Kaliumhydroxid, oder einer Mineralsäure, z.B. von Salzsäure, Schwefelsäure oder Phosphorsäure, in die freie Carboxylgruppe R oder z.B. durch Umsetzung mit Ammoniak oder dem entsprechenden, mindestens

ein Wasserstoffatom aufweisenden Amin in eine amidierte Carboxylgruppe R überführt werden.

Eine veresterte Carboxylgruppe R kann ferner in üblicher Weise, z.B. durch Umsetzung mit einem Metallsalz, wie dem Natrium- oder Kaliumsalz, eines entsprechenden Alkohols oder mit diesem selbst in Gegenwart eines Katalysators, beispielsweise einer starken Base, z.B. von Natrium- oder Kaliumhydroxid, oder einer starken Säure, wie einer Mineralsäure, z.B. von Salzsäure, Schwefelsäure oder Phosphorsäure, oder einer organischen Sulfonsäure, z.B. von p-Toluolsulfonsäure, oder einer Lewissäure, z.B. von Bortrifluorid-Aetherat, zu einer anderen veresterten Carboxylgruppe R umgeestert werden.

Eine amidierte Carboxylgruppe R kann in üblicher Weise, z.B. durch Hydrolyse in Gegenwart eines Katalysators, beispielsweise einer starken Base, wie eines Alkalimetall- oder Erdalkalimetallhydroxides oder -carbonates, z.B. von Natrium- oder Kaliumhydroxid oder -carbonat, oder einer starken Säure, wie einer Mineralsäure, z.B. von Salzsäure, Schwefelsäure oder Phosphorsäure, in die freie Carboxylgruppe R umgewandelt werden.

Am Rest Ph einer erfindungsgemäss erhältlichen Verbindung kann man weiterhin gegebenenfalls veresterte oder verätherte Hydroxygruppen ineinander umwandeln.

So kann man beispielsweise eine freie Hydroxylgruppe durch Umsetzung mit einem Verätherungsmittel, z.B. mit einem Niederalkylierungsmittel, zu einer verätherten Hydroxygruppe, z.B. einer Niederalkoxy-, Hydroxyniederalkoxy- oder Niederalkylendioxygruppe veräthern.

Veräthernde Mittel sind beispielsweise reaktionsfähige veresterte Alkohole, wie mit einer Mineralsäure, z.B. mit Jod-, Chlor- oder Bromwasserstoff- oder Schwefelsäure, oder organischen Sulfonsäure, z.B. mit p-Toluol-, p-Brombenzol-, Benzol-, Methan-, Aethan- oder Aethensulfonsäure, oder Fluorsulfonsäure veresterte Niederalkanole oder Niederalkandiole, sowie Diazoniederalkane. Als veräthernde Mittel sind insbesondere Niederalkylchloride, -bromide oder -jodide, z.B. Methyljodid, Niederalkylenhalohydrine, z.B. Aethylenchlorhydrin, Diniederalkylsulfate, z.B. Dimethyl- oder Diäthylsulat, oder Methylfluorsulfonat, Niederalkylsulfonate, wie Methyl- oder Aethyl-methan-, -p-toluol- oder p-brombenzolsulfonate, Epoxyniederalkane, z.B. Propylenoxid, sowie Diazoalkane, z.B. Diazomethan, zu nennen.

Die Umsetzungen mit Verätherungsmitteln, z.B. den vorstehend hervorgehobenen, kann in üblicher Weise durchgeführt werden, bei der Umsetzung mit einem Diazoniederalkan z.B. in einem inerten Lösungsmittel, wie einem Aether, z.B. in Tetrahydrofuran, oder bei der Verwendung eines reaktionsfähigen veresterten Alkohols beispielsweise in Gegenwart eines basischen Kondensationsmittels, wie einer anorganischen Base, z.B. von Natrium-, Kalium- oder Calciumhydroxid oder -carbonat, oder einer tertiären oder quaternären Stickstoffbase, z.B. von Pyridin, Triäthylamin, oder Tetraäthyl- oder Benzyltriäthylammoniumhydroxid, und/oder eines für die jeweilige Umsetzung üblichen Lösungsmittels, welches auch aus einem Ueberschuss des für die Verätherung beispielsweise verwendeten Niederalkylhalogenides oder -sulfates, und/oder einer als basisches Kondensationsmittel verwendeten tertiären Stickstoffbase, z.B. Triäthylamin oder Pyridin, bestehen kann, erforderlichenfalls bei erhöhter Temperatur. Empfehlenswert ist insbesondere die Methylierung mittels Methyljodid im Amylalkohol/Kaliumcarbonat bei Siedetemperatur.

Umgekehrt kann man veräthertes Hydroxy in üblicher Weise, beispielsweise in Gegenwart eines sauren Mittels, wie einer Halogenwasserstoffsäure, z.B. von Jodwasserstoffsäure, in einem inerten Lösungsmittel, z.B. in Aethanol oder Essigsäure, in Hydroxy umwandeln.

In analoger Weise kann man auch verestertes Hydroxy, wie Halogen, durch Umsetzung mit einem entsprechenden Metallalkoholat, wie einem Alkalimetallniederalkanolat, z.B. mit Natriummethanolat, in veräthertes Hydroxy umwandeln.

Ferner kann man in einer erfindungsgemäss erhältlichen Verbindung der Formel IV, Glykoloyl $X_1$ durch Umsetzung mit einem veresternden Mittel, wie einem Niederalkansäure- oder Benzoesäureanhydrid oder -chlorid, verestern oder durch Ueberführung in ein Alkalimetallsalz und Umsetzung mit einer entsprechenden Halogenverbindung, wie einem Niederalkylhalogenid, veräthern. Umgekehrt kann man verestertes oder veräthertes Glykoloyl, z.B. säurekatalytisch, zu Glykoloyl hydrolysieren.

Erfindungsgemäss erhältliche freie Verbindungen der Formel I, worin R für Carboxy steht, können in an sich bekannter Weise in Salze übergeführt werden, u.a. durch Behandeln mit einer Base oder mit einem geeigneten Salz einer Carbonsäure, üblicherweise in Gegenwart eines Lösungs- oder Verdünnungsmittels.

Erfindungsgemäss erhältliche Salze können in an sich bekannter Weise in die freien Verbindungen umgewandelt werden, z.B. durch Behandlen mit einem sauren Reagens, wie einer Mineralsäure.

Die Verbindungen einschliesslich ihrer Salze können auch in Form ihrer Hydrate erhalten werden oder das zur Kristallisation verwendete Lösungsmittel einschliessen.

Infolge der engen Beziehung zwischen den neuen Verbindungen in freier Form und in Form ihrer Salze sind im

vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes und/oder Racemates bzw. Antipoden verwendet oder insbesondere unter den Reaktionsbedingungen bildet.

Vorzugsweise verwendet man jeweils solche Ausgangsstoffe, die zu den eingangs als bevorzugten Erfindungsgegenstand bezeichneten Zielverbindungen führen.

Die vorliegende Erfindung betrifft ebenfalls pharmazeutische Präparate, welche Verbindungen der Formel (I) oder pharmazeutisch verwendbare Salze davon enthalten. Bei den erfindungsgemässen pharmazeutischen Präparaten handelt es sich um solche, die zur topischen und lokalen sowie enteralen, wie oralen oder rektalen, sowie parenteralen Verabreichung an und zur Inhalation durch Warmblüter, bestimmt sind und den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab.

Die neuen pharmazeutischen Präparate enthalten z.B. von etwa 10% bis etwa 95%, vorzugsweise von etwa 20% bis etwa 90% des Wirkstoffs. Erfindungsgemässe pharmazeutische Präparate sind z.B. solche Aerosol- oder Sprayform oder in Dosiseinheitsformen, wie Dragées, Tabletten, Kapseln oder Suppositorien, ferner Ampullen.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, z.B. von Mais-, Weizen-, Reis- oder Kartoffelstärkekleister, Gelatine, Traganth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaftresistenten Ueberzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephathalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere, oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enhalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel

oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natrium-carboxymethylcellulose, Sorbit und/oder Dextran und gegebenenfalls auch Stabilisatoren enthalten.

Inhalationspräparate für die Behandlung der Atemwege durch nasale oder buccale Verabreichung sind z.B. Aerosole oder Sprays, welche den pharmakologischen Wirkstoff in Form eines Puders oder in Form von Tropfen einer Lösung oder Suspension verteilen können. Präparate mit Puder-verteilenden Eigenschaften enthalten ausser dem Wirkstoff üblicherweise ein flüssiges Treibgas mit einem Siedepunkt unter der Raumtemperatur, sowie, wenn erwünscht, Trägerstoffe, wie flüssige oder feste nicht-ionische oder anionische oberflächenaktive Mittel und/oder feste Verdünnungsmittel. Präparate, in welchen der pharmakologische Wirkstoff in Lösung vorliegt, enthalten ausser diesem ein geeignetes Treibmittel, ferner, falls notwenidig, ein zusätzliches Lösungsmittel und/oder einen Stabilisator. Anstelle des Treibgases kann auch Druckluft verwendet werden, wobei diese mittels einer geeigneten Verdichtungs- und Entspannungsvorrichtung nach Bedarf erzeugt werden kann.

Pharmazeutische Präparate für topische und lokale Verwendung sind z.B. für die Behandlung der Haut Lotionen und Cremen, die eine flüssige oder semifeste Oel-in-Wasser- oder Wasser-in-Oel-Emulsion enthalten, und Salben (wobei solche vorzugsweise ein Konservierungsmittel enthalten), für die Behandlung der Augen Augentropfen, welche die aktive Verbindung in wässriger oder öliger Lösung enthalten und Augensalben, die vorzugsweise in steriler Form hergestellt werden, für die Behandlung der Nase Puder, Aerosole und Sprays (ähnlich

den oben beschriebenen für die Behandlung der Atemwege), sowie grobe Puder, die durch schnelles Inhalieren durch die Nasenlöcher verabreicht werden, und Nasentropfen, welche die aktive Verbindung in wässriger oder öliger Lösung enthalten, oder für die lokale Behandlung des Mundes Lutschbonbons, welche die aktive Verbindung in einer im allgemeinen aus Zucker und Gummiarabikum oder Tragakanth gebildeten Masse enthalten, welcher Geschmacksstoffe beigegeben sein können, sowie Pastillen, die den Aktivstoff in einer inerten Masse, z.B. aus Gelatine und Glycerin oder Zucker und Gummiarabikum, enthalten.

Die Erfindung betrifft ebenfalls die Verwendung der neuen Verbindungen der Formel (I) und ihrer Salze als pharmakologisch aktive Verbindungen, insbesondere als Antiallergika, vorzugsweise in der Form von pharmazeutischen Präparaten. Die tägliche Dosis, die einem Warmblüter von etwa 70 kg verabreicht wird, beträgt von etwa 200 mg bis etwa 1200 mg.

Die nachfolgenden Beispiele illustrieren die oben beschriebene Erfindung; sie solle jedoch diese in ihrem Umfang in keiner Weise einschränken. Temperaturen werden in Celsiusgraden angegeben.

**Beispiel 1**

Zu einer Lösung von 3,7 g Triäthylamin und 5 g 3-Amino-4-oxo-4H-1-benzopyran in 100 ml Chloroform fügt man unter Rühren in einer wasserfreien Atmosphäre bei 10° tropfenweise 4,5 g Oxalsäuremonomethylesterchlorid hinzu. Nach beendeter Zugabe lässt man 90 Minuten bei Raumtemperatur nachrühren. Dann verteilt man die Reaktionslösung zwischen 3-mal 50 ml Chloroform und 50 ml 2n-Salzsäure. Die organischen Phasen werden neutralgewaschen, über Natriumsulfat getrocknet und im Vakuum eingeengt. Nach Zugabe von Aether zur konzentrierten Lösung kristallisiert das 3-Methoxyoxalylamino-4-oxo-4H-1-benzopyran vom F. 200-201° aus.

**Beispiel 2**

Zu einer Suspension von 7,6 g 3-Methoxyoxalylamino-4-oxo-4H-1-benzopyran in 200 ml Aethanol fügt man 35 ml n-Natronlauge und anschliessend 1,3 l Wasser hinzu. Nun erwärmt man unter Rühren 15 Minuten auf 70°. Die nunmehr klare Lösung wird mit konzentrierter Salzsäure auf $P_H$=2 gestellt und langsam auf Raumtemperatur abgekühlt. Dabei kristallisiert das 3-Oxaloamino-4-oxo-4H-1-benzopyran vom F. 200° (Zers.) aus.

**Beispiel 3**

In analoger Weise wie in Beispiel 1 bzw. 2 beschrieben kann man ferner herstellen:

6-Hydroxy-3-methoxyoxalylamino-4-oxo-4H-1-benzopyran,

6-Hydroxy-3-oxaloamino-4-oxo-4H-1-benzopyran,

6-Chlor-3-methoxyoxalylamino-4-oxo-4H-1-benzopyran,

6-Chlor-3-oxaloamino-4-oxo-4H-1-benzopyran, F. 213-215°,

6-Methoxy-3-methoxyoxalylamino-4-oxo-4H-1-benzopyran, F. 174-175°,

6-Methoxy-3-oxaloamino-4-oxo-4H-1-benzopyran, F. 230°,

5,8-Dimethyl-3-methoxyoxalylamino-4-oxo-4H-1-benzopyran,
5,8-Dimethyl-3-oxaloamino-4-oxo-4H-1-benzopyran,
3-Methoxyoxalylamino-6,7-trimethylen-4-oxo-4H-1-benzopyran, F. 185-186° und
3-Oxaloamino-6,7-trimethylen-4-oxo-4H-1-benzopyran, F. 185°.

Beispiel 4

Zu einer Lösung von 1,95 g 3-Amino-6-chlor-4-oxo-4H-1-benzopyran in 1,58 g Pyridin und 100 ml Methylenchlorid werden tropfenweise 1,36 g Oxalsäuremonoäthylesterchlorid hinzugefügt. Die Reaktionslösung wird 90 Minuten bei 90° gerührt und unter vermindertem Druck zur Trockne eingedampft. Der gelbliche, feste Eindampfrückstand wird mit Wasser digeriert, abfiltriert, getrocknet und aus Aethanol umkristallisiert. Man erhält das 3-Aethoxyoxalylamino-6-chlor-4-oxo-4H-1-benzopyran vom F. 161-162°.

Beispiel 5

In analoger Weise wie in Beispiel 4 beschrieben erhält man ausgehend von 20 g 3-Amino-4-oxo-4H-1-benzopyran das 3-Aethoxyoxalylamino-4-oxo-4H-1-benzopyran vom F. 146,5-148°.

Beispiel 6

In analoger Weise wie in Beispiel 4 beschrieben erhält man ausgehend von 2,0 g 3-Amino-2-methyl-4-oxo-4H-1-benzopyran das 3-Aethoxyoxalylamino-2-methyl-4-oxo-4H-1-benzopyran vom F. 100-104°.

Das Ausgangsmaterial kann z.B. folgendermassen hergestellt werden:

Eine Lösung von 5,0 g 2-Methyl-3-nitro-4-oxo-4H-1-benzopyran in 250 ml Aethanol wird mit 0,75 g 10%-iger Palladiumkohle versetzt und bei Raumtemperatur und Normaldruck 40 Minuten lang hydriert. Der Katalysator wird abfiltriert und das Lösungsmittel unter vermindertem Druck abgezogen. Der gelbe Eindampfrückstand wird aus Benzol umkristallisiert. Man erhält das 3-Amino-2-methyl-4-oxo-4H-1-benzopyran vom F. 118-120°.

Beispiel 7

Zu einer Lösung von 2,7 g 3-Aethoxyoxalylamino-6-chlor-4-oxo-4H-1-benzopyran in 200 ml 50%-igem wässrigen Aethanol wird unter gelegentlichem Erwärmen auf einem Dampfbad bis zur schwach basischen Reaktion tropfenweise 0,1-n Natronlauge hinzugefügt. Die Farbe der Reaktionsmischung schlägt zunächst nach Orange und dann nach Weiss um. Es wird weitere 0,1n-Natronlauge hinzugefügt, bis eine schwache Orangefärbung bestehen bleibt. Der Niederschlag wird abfiltriert, mit n-Salzsäure und anschliessend mit Wasser gewaschen, abgesaugt, getrocknet und aus Methanol umkristallisiert. Man erhält 6-Chlor-3-oxaloamino-4-oxo-4H-1-benzopyran vom F. 213-215°.

Beispiel 8

In analoger Weise wie in Beispiel 7 beschrieben erhält man ausgehend von 15,5 g 3-Aethoxyoxalylamino-4-oxo-4H-1-benzopyran das 3-Oxaloamino-4-oxo-4H-1-benzopyran vom F. 195-197° (aus Aethanol) und ausgehend von 1 g 3-Aethoxyoxalylamino-2-methyl-4-oxo-4H-1-benzopyran das 2-Methyl-3-oxaloamino-4-oxo-4H-1-benzopyran vom F. 208-209° (aus Aethanol).

Beispiel 9

In analoger Weise wie in Beispiel 4 beschrieben erhält man ausgehend von 5 g 3-Amino-7-methoxy-4-oxo-4H-1-benzopyran das 3-Aethoxyoxalylamino-7-methoxy-4-oxo-4H-1-benzopyran vom F. 218-220° (aus Essigsäureäthylester).

Das Ausgangsmaterial kann z.B. folgendermassen hergestellt werden:
1,85 g 4-Hydroxy-7-methoxy-3-nitro-cumarin werden in 60 ml 5%-iger wässriger Natronlauge 20 Stunden bei Raumtemperatur gerührt. Die Reaktionsmischung wird abfiltriert, trockengesaugt und aus Aethanol umkristallisiert. Man erhält das 2-Hydroxy-4-methoxy-β-nitro-acetophenon vom F. 138-140°.

2,11 g 2-Hydroxy-4-methoxy-β-nitro-acetophenon werden zu einem gerührten Gemisch von 0,68 g Natriumformat und 25 ml des gemischten Anhydrides von Essig- und Ameisensäure hinzugefügt. Man erwärmt auf 80°, lässt auf Raumtemperatur abkühlen, rührt 1 Stunde bei Raumtemperatur nach, fügt 100 ml Eiswasser hinzu, saugt ab, wäscht mit Wasser nach, saugt trocken und kristallisiert aus Essigsäureäthylester um. Man erhält 7-Methoxy-3-nitro-4-oxo-4H-1-benzopyran vom F. 185-187°.

Zu einer unter Stickstoff gerührten Suspension von 7 g 7-Methoxy-3-nitro-4-oxo-4H-1-benzopyran in 80 ml Aethanol und 30 ml Wasser werden 20 g Natriumdithionit hinzufügt. Man lässt 20 Minuten rühren, dampft unter vermindertem Druck ein, löst den festen Eindampfrückstand in Wasser und extrahiert mit Chloroform. Der Chloroformauszug wird über Natriumsulfat getrocknet und eingedampft. Man erhält das 3-Amino-7-methoxy-4-oxo-4H-1-benzopyran vom F. 143-146°.

## Beispiel 10

In analoger Weise wie in Beispiel 7 beschrieben erhält man ausgehend von 2,1 g 3-Aethoxyoxalylamino-7-methoxy-4-oxo-4H-1-benzopyran das 7-Methoxy-3-oxaloamino-4-oxo-4H-1-benzopyran vom F. 236-238° (aus Aethanol).

## Beispiel 11

In analoger Weise wie in Beispiel 1 beschrieben kann man ausgehend von 3-Amino-5,7-dimethyl-4-oxo-4H-1-benzopyran das 5,7-Dimethyl-3-methoxyoxalylamino-4-oxo-4H-1-benzopyran vom F. 186-187° (aus Aethanol) herstellen. Das Ausgangsmaterial kann z.B. ausgehend von 5,7-Dimethyl-4-hydroxy-cumarin durch Umsetzung mit Salpetersäure in Essigsäure bei 80° zum 5,7-Dimethyl-4-hydroxy-3-nitro-cumarin, 24-stündiger Behandlung desselben mit Natronlauge und auschliessendem Ansäurern unter Decarboxylierung zu 4,6-Dimethyl-2-hydroxy-β-nitro-acetophenon in Analogie zu dem in J. Am. Chem. Soc. 67, 99 (1945) beschriebenen Verfahren sowie Umsetzung desselben mit dem gemischten Anhydrid von Ameisen- und Essigsäure zu 5,7-Dimethyl-3-nitro-4-oxo-4H-1-benzopyran vom F. 120-125° und Reduktion desselben mit Natriumdithionit zum 3-Amino-5,7-Dimethyl-4-oxo-4H-1-benzopyran (F. 120-121°) in Analogie zu dem in Tetrahedron Letters 1976, 719 beschriebenen Verfahren und Reinigung desselben über den Hydrochlorid erhalten werden.

Beispiel 12

In Analogie zu dem in Beispiel 11 beschriebenen Verfahren erhält man ausgehend 4-Hydroxy-8-methyl-cumarin über 4-Hydroxy-8-methyl-3-nitro-cumarin von F. 178-180° (Zers.), 2-Hydroxy-3-methyl-β-nitro-acetophenon vom F. 126-128° (aus Aethanol), 8-Methyl-3-nitro-4-oxo-4H-1-benzopyran vom F. 85-95° und 3-Amino-8-methyl-4-oxo-4H-1-benzopyran vom F. 151-152° das 3-Methoxyoxalylamino-8-methyl-4-oxo-4H-1-benzopyran vom F. 164,5°.

Beispiel 13

In analoger Weise wie in Beispiel 11 beschrieben erhält man ausgehend von 4-Hydroxy-6-methoxy-cumarin über 4-Hydroxy-6-methoxy-3-nitro-cumarin vom F. 172-174°, 2-Hydroxy-5-methoxy-β-nitro-acetophenon vom F. 143-144° (aus Methylenchlorid/Petroläther), 6-Methoxy-3-nitro-4-oxo-4H-1-benzopyran vom F. 149-150° und 3-Amino-6-methoxy-4-oxo-4H-1-benzopyran das 6-Methoxy-3-methoxyoxalyl-amino-4-oxo-4H-1-benzopyran vom F. 174-175° (aus Aethanol/Chloroform).

Beispiel 14

In analoger Weise wie in Beispiel 11 beschrieben erhält man ausgehend von 6,7-Dimethyl-4-hydroxy-cumarin über 6,7-Dimethyl-4-hydroxy-3-nitro-cumarin vom F. 205-207° (Zers.), 4,5-Dimethyl-2-hydroxy-β-nitro-acetophenon vom F. 142-144° (aus Methylenchlorid/Petroläther), 6,7-Dimethyl-3-nitro-4-oxo-4H-1-benzopyran vom F. 155-157° und 3-Amino-6,7-dimethyl-4-oxo-4H-1-benzopyran vom F. 150-152° (aus Chloroform/Toluol) das 6,7-Dimethyl-3-methoxyoxalylamino-4-oxo-4H-1-benzopyran vom F. 222-224° (aus Chloroform/Aethanol).

Beispiel 15

In analoger Weise wie in Beispiel 11 beschrieben erhält man ausgehend von 4-Hydroxy-6-methyl-cumarin über 4-Hydroxy-6-methyl-3-nitro-cumarin vom F. 178° (Zers.), 2-Hydroxy-5-methyl-β-nitro-acetophenon vom F. 137-138° (aus Aethanol), 6-Methyl-3-nitro-4-oxo-4H-1-benzopyran vom F. 97-99° und 3-Amino-6-methyl-4-oxo-4H-1-benzopyran vom F. 96-98° (aus Essigsäureäthylester/Petroläther) das 3-Methoxyoxalylamino-6-methyl-4-oxo-4H-1-benzopyran vom F. 179,5° (aus Chloroform/Aethanol).

Beispiel 16

In analoger Weise wie in Beispiel 11 beschrieben erhält man ausgehend von 4-Hydroxy-4,6,7,8-tetrahydro-2-oxo-cyclopenta[g]-1-benzopyran über 4-Hydroxy-3-nitro-4,6,7,8-tetrahydro-cumarin vom F. 215-217° (Zers.), 5-(2-Nitroacetyl)-6-hydroxy-indan vom F. 115-117° (aus Methylenchlorid/Petroläther), 3-Nitro-4-oxo-4,6,7,8-tetrahydro-cyclopenta[g]-1-benzopyran vom F. 145° (Zers.) und 3-Amino-4-oxo-4,6,7,8-tetrahydro-cyclopenta[g]-1-benzopyran vom F. 151-152° (aus Essigsäureäthylester/Petroläther) das 3-Methoxyoxalylamino-4-oxo-4,6,7,8-tetrahydro-cyclopenta[g]-1-benzopyran der Formel vom F. 185-186°.

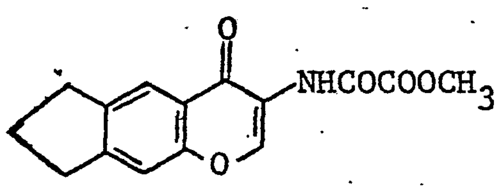

Beispiel 17

In analoger Weise wie in Beispiel 2 beschrieben erhält man

5,7-Dimethyl-3-oxaloamino-4-oxo-4H-1-benzopyran vom F. 224 (Zers., aus Wasser/Aethanol), ausgehend von 5,7-Dimethyl-3-methoxyoxalylamino-4-oxo-4H-1-benzopyran,

6,7-Dimethyl-3-oxaloamino-4-oxo-4H-1-benzopyran vom F. 205° (aus Wasser/Aethanol), ausgehend von 6,7-Dimethyl-3-methoxyoxalylamino-4-oxo-4H-1-benzopyran,

6-Methoxy-3-oxaloamino-4-oxo-4H-1-benzopyran vom F. 230° (aus Wasser/Aethanol), ausgehend von 6-Methoxy-3-methoxyoxalylamino-4-oxo-4H-1-benzopyran,

6-Methyl-3-oxaloamino-4-oxo-4H-1-benzopyran vom F. 199,5° (aus Wasser/Aethanol), ausgehend von 3-Methoxyoxalylamino-6-methyl-4-oxo-4H-1-benzopyran und

3-Oxaloamino-4-oxo-4,6,7,8-tetrahydro-cyclopenta[g]-1-benzopyran vom F. 185° (Zers.), ausgehend von 3-Methoxy-oxalylamino-4-oxo-4,6,7,8-tetrahydro-cyclopenta[g]-1-benzopyran.

Beispiel 18

Einer Suspension von 9 g 3-Amino-4-oxo-4H-1-benzopyran in 3,6 g Oxalsäuredimethylester und 300 ml Xylol wird unter Rühren in einer Stickstoffatmosphäre zum Sieden erhitzt. Innert 24 Stunden werden ca. 200 ml Xylol abdestilliert. Nun lässt man auf 90° abkühlen und filtriert den gebildeten Niederschlag eines Nebenprodukts ab und dampft zum Trockne ein. Aus dem Eindampfrückstand erhält man aus Aethanol/Petroläther das 3-Methoxyoxalylamino-4-oxo-4H-1-benzopyran vom F. 200-201°.

Beispiel 19

In analoger Weise wie in den Beispielen 1 bis 18 beschrieben erhält man ferner

6-Chlor-3-methoxyoxalylamino-7-methyl-4-oxo-4H-1-benzopyran,
6-Chlor-7-methyl-3-oxaloamino-4-oxo-4H-1-benzopyran,
6-Chlor-3-methoxyoxalylamino-8-methyl-4-oxo-4H-1-benzopyran und
6-Chlor-8-methyl-3-oxaloamino-1-benzopyran.

Beispiel 20

Tabletten, enthaltend 0,1 g 3-Methoxyoxalylamino-4-oxo-4H-1-benzopyran, werden wie folgt hergestellt:

Zusammensetzung (für 1000 Tabletten):

| | |
|---|---|
| 3-Methoxyoxalylamino-4-oxo-4H-1-benzopyran | 100 g |
| Lactose | 50 g |
| Weizenstärke | 73 g |
| Kolloidale Kieselsäure | 13 g |
| Magnesiumstearat | 2 g |
| Talk | 12 g |
| Wasser | q.s. |

Das 3-Methoxyoxalylamino-4-oxo-4H-1-benzopyran wird mit einem Teil der Weizenstärke, mit der Lactose und der kolloidalen Kieselsäure vermischt und das Gemisch durch ein Sieb getrieben. Ein weiterer Teil der Weizenstärke wird mit der fünffachen Menge Wasser auf dem Wasserbad verkleistert und die obige Pulvermischung mit diesem Kleister angeknetet, bis eine schwach plastische Masse entstanden ist. Die plastische Masse wird durch ein Sieb von etwa 3 mm Maschenweite gedrückt, getrocknet und das trockene Granulat nochmals durch ein Sieb getrieben. Darauf werden die restliche Weizenstärke, der Talk und das Magnesiumstearat zugemischt und die erhaltene Mischung zu Tabletten von 0,25 g verpresst.

In analoger Weise können auch Tabletten enthaltend jeweils 0,1 g
3-Oxaloamino-4-oxo-4H-1-benzopyran,
6-Hydroxy-3-methoxyoxalylamino-4-oxo-4H-1-benzopyran,
6-Hydroxy-3-oxaloamino-4-oxo-4H-1-benzopyran,
6-Chlor-3-methoxyoxalylamino-4-oxo-4H-1-benzopyran,
6-Chlor-3-oxaloamino-4-oxo-4H-1-benzopyran,
6-Methoxy-3-methoxyoxalylamino-4-oxo-4H-1-benzopyran,
6-Methoxy-3-oxaloamino-4-oxo-4H-1-benzopyran,
5,8-Dimethyl-3-methoxyoxalylamino-4-oxo-4H-1-benzopyran,
5,8-Dimethyl-3-oxaloamino-4-oxo-4H-1-benzopyran,
3-Methoxyoxalylamino-6,7-trimethylen-4-oxo-4H-1-benzopyran und
3-Oxaloamino-6,7-trimethylen-4-oxo-4H-1-benzopyran
hergestellt werden.

## Beispiel 21

Eine zur Inhalation geeignete, etwa 2%-ige wässrige Lösung eines in freier Form oder in Form des Natriumsalzes wasserlöslichen erfindungsgemässen Wirkstoffes kann z.B. in folgender Zusammensetzung hergestellt werden:

### Zusammensetzung

| | | |
|---|---|---|
| Wirkstoff, z.B. 3-Oxaloamino-4-oxo-4H-1-benzopyran | | 2000 mg |
| Stabilisator, z.B. Aethylendiamintetraessigsäure-dinatriumsalz | | 10 mg |
| Konservierungsmittel, z.B. Benzalkoniumchlorid | | 10 mg |
| Wasser, frisch destilliert | ad | 100 ml |

### Herstellung

Der Wirkstoff wird unter Zusatz der äquimolekularen Menge 2n-Natronlauge in frisch destilliertem Wasser gelöst. Dann wird der Stabilisator und das Konservierungsmittel hinzugegeben.

Nach vollständiger Auflösung aller Komponenten wird die erhaltene Lösung auf 100 ml aufgefüllt, in Fläschchen abgefüllt und diese gasdicht verschlossen.

In analoger Weise kann man auch 2%-ige wässrige Inhalationslösungen enthaltend
3-Methoxyoxalylamino-4-oxo-4H-1-benzopyran
6-Hydroxy-3-methoxyoxalylamino-4-oxo-4H-1-benzopyran,
6-Hydroxy-3-oxaloamino-4-oxo-4H-1-benzopyran,
6-Chlor-3-methoxyoxalylamino-4-oxo-4H-1-benzopyran,
6-Chlor-3-oxaloamino-4-oxo-4H-1-benzopyran,
6-Methoxy-3-methoxyoxalylamino-4-oxo-4H-1-benzopyran,
6-Methoxy-3-oxaloamino-4-oxo-4H-1-benzopyran,
5,8-Dimethyl-3-methoxyoxalylamino-4-oxo-4H-1-benzopyran,
5,8-Dimethyl-3-oxaloamino-4-oxo-4H-1-benzopyran,
3-Methoxyoxalylamino-6,7-trimethylen-4-oxo-4H-1-benzopyran und
3-Oxaloamino-6,7-trimethylen-4-oxo-4H-1-benzopyran
herstellen.

## Beispiel 22

Zur Insufflation geeignete, etwa 25 mg eines erfindungsgemässen Wirkstoffes enthaltende Kapseln können z.B. folgendermassen hergestellt werden:

### Zusammensetzung

| | |
|---|---|
| Wirkstoff, z.B. 3-Methoxyoxalylamino-4-oxo-4H-1-benzopyran | 25 mg |
| Lactose, feinst gemahlen .. | 25 mg |

### Herstellung

Der Wirkstoff und die Lactose werden innig vermischt. Das erhaltene Pulver wird sodann gesiebt und in Portionen zu je 50 mg in 1000 Gelatinekapseln abgefüllt.

In analoger Weise kann man auch Insufflationskapseln enthaltend jeweils 25 mg
3-Oxaloamino-4-oxo-4H-1-benzopyran,
6-Hydroxy-3-methoxyoxalylamino-4-oxo-4H-1-benzopyran,
6-Hydroxy-3-oxaloamino-4-oxo-4H-1-benzopyran,
6-Chlor-3-methoxyoxalylamino-4-oxo-4H-1-benzopyran,
6-Chlor-3-oxaloamino-4-oxo-4H-1-benzopyran,
6-Methoxy-3-methoxyoxalylamino-4-oxo-4H-1-benzopyran,
6-Methoxy-3-oxaloamino-4-oxo-4H-1-benzopyran,
5,8-Dimethyl-3-methoxyoxalylamino-4-oxo-4H-1-benzopyran,
5,8-Dimethyl-3-oxaloamino-4-oxo-4H-1-benzopyran,
3-Methoxyoxalylamino-6,7-trimethylen-4-oxo-4H-1-benzopyran und
3-Oxaloamino-6,7-trimethylen-4-oxo-4H-1-benzopyran
herstellen.

## Beispiel 23

In analoger Weise wie in den Beispielen 20-22 beschrieben können pharmazeutische Präparate enthaltend
3-Aethoxyoxalylamino-6-chlor-4-oxo-4H-1-benzopyran,
N-(6,7-Dimethyl-4-oxo-4H-benzopyran-3-yl)-oxamid,
2-Methyl-3-oxaloamino-4-oxo-4H-1-benzopyran,
7-Methoxy-3-oxaloamino-4-oxo-4H-1-benzopyran,
5,7-Dimethyl-3-methoxyoxalylamino-4-oxo-4H-1-benzopyran,
3-Methoxyoxalylamino-8-methyl-4-oxo-4H-1-benzopyran,
6,7-Dimethyl-3-methoxyoxalylamino-4-oxo-4H-1-benzopyran,
5,7-Dimethyl-3-oxaloamino-4-oxo-4H-1-benzopyran,
6,7-Dimethyl-3-oxaloamino-4-oxo-4H-1-benzopyran
3-Methoxyoxalylamino-4-oxo-2,6,7-trimethyl-4H-1-benzopyran,
3-Oxaloamino-4-oxo-2,6,7-trimethyl-4H-1-benzopyran,
3-Glycoloylamino-4-oxo-4,6,7,8-tetrahydro-cyclopenta[g]-1-benzopyran,
3-Acetoxyglycoloylamino-4-oxo-4,6,7,8-tetrahydro-cyclopenta[g]-1-benzopyran und
6-Methyl-3-oxaloamino-4-oxo-4H-1-benzopyran
als Wirkstoff hergestellt werden.

Beispiel 24

Zu einer Lösung von 0,2 g 6,7-Dimethyl-3-methoxy-oxalylamino-4-oxo-4H-1-benzopyran in 30 ml Methanol und 20 ml Methylenchlorid werden 15 ml gesättigter methanolischer Ammoniaklösung hinzugefügt. Das ausfallende N-(6,7-Dimethyl-4-oxo-4H-1-benzopyran-3-yl)-oxamid wird abfiltriert, mit wenig Methanol gewaschen und getrocknet; es schmilzt bei 280-282°.

Beispiel 25

Zu einem auf 60° erwärmten Gemisch von 50 ml Aethanol und 0,5 ml 2n-Natronlauge werden 0,5 g 3-Chlor-oxalylamino-5,7-dimethyl-4-oxo-4H-1-benzopyran hinzugefügt. Man versetzt mit 300 ml Wasser, erwärmt auf 60° und säuert dann mit 2n-Salzsäure auf pH=1 an. Der ausfallende Niederschlag wird abfiltriert, mit wenig Aethanol gewaschen und getrocknet. Man erhält das 5,7-Dimethyl-3-oxaloamino-4-oxo-1-benzopyran vom Smp. 222° (Zers.).

Das Ausgangsmaterial kann folgendermassen hergestellt werden:

Zu 5 ml Oxalylchlorid wird unter Stickstoff langsam die Lösung von 0,5 g 3-Amino-5,7-dimethyl-4-oxo-4H-1-benzopyran in 5 ml Chloroform zugetropft. Man lässt eine Stunde nachrühren, dampft unter vermindertem Druck zu Trockne ein, löst den Eindampfruckstand in 30 ml Chloroform, filtriert und dampft erneut zur Trockne ein. Man erhält das 3-Chloroxalylamino-5,7-dimethyl-4-oxo-4H-1-benzopyran vom Smp. 150-153°.

Beispiel 26

Zu einer Lösung von 0,2 g 3-Glycoloylamino-4-oxo-4,6,7,8-tetrahydro-cyclopenta[g]-1-benzopyran in 40 ml Aceton und 40 ml Wasser fügt man 0,2 g Kaliumpermanganat hinzu

und lässt 40 Stunden bei Raumtemperatur rühren. Man filtriert den gebildeten Niederschlag ab, säuert das Filtrat mit 2n-Salzsäure an und filtriert den ausgeschiedenen Niederschlag ab. Man erhält so 3-Oxaloamino-4-oxo-4,6,7,8-tetrahydro-cyclopenta[g]-1-benzopyran vom Smp. 185° (Zers.).

Das Ausgangsmaterial kann z.B. folgendermassen hergestellt werden:

Zu 6 g 3-Amino-4-oxo-4,6,7,8-tetrahydro-cyclopenta[g]-1-benzopyran werden 7 g Glycolsäure hinzugefügt. Die Reaktionsmischung wird 1 Stunde auf 120° erwärmt. Man lässt auf Raumtemperatur abkühlen, digeriert mit Wasser, filtriert ab, trocknet und kristallisiert aus 400 ml Aethanol und anschliessend aus wenig Essigsäureäthylester um. Man erhält das 3-Glycoloylamino-4-oxo-4,6,7,8-tetrahydro-cyclopenta[g]-1-benzopyran vom Smp. 199-200°.

Aus diesem kann man durch übliche Acetylierung mit Acetanhydrid das 3-Acetoxyglycoloylamino-4-oxo-4,6,7,8-tetrahydro-cyclopenta[g]-1-benzopyran vom Smp. 199-200° herstellen.

Beispiel 27

Durch Umsetzung von 3-Oxaloamino-4-oxo-4H-1-benzopyran mit der äquimolekularen Menge 2n-Natronlauge erhält man das Natriumsalz und durch Umsetzung der genannten Säure, gelöst in heissem Dimethylformamid mit 0,1 n. Calciumchloridlösung das Calciumsalz des 3-Oxaloamino-4-oxo-4H-1-benzopyrans, welche bis 300° nicht schmelzen.

Beispiel 28

In analoger Weise wie in Beispiel 1 beschrieben erhält man durch Umsetzung von 3-Amino-4-oxo-2,6,7-trimethyl-4H-1-benzopyran mit Oxalsäuremethylesterchlorid das 3-Methoxyoxalylamino-4-oxo-2,6,7-trimethyl-4H-1-benzopyran vom

Smp. 210-211°. Das Ausgangsmaterial kann in üblicher Weise ausgehend von 6,7-Dimethyl-4-hydroxy-3-nitro-cumarin durch behandlung mit Natronlauge und saure Decarboxylierung zum 2-Hydroxy-4,5-dimethyl-β-nitro-acetophenon, Umsetzung derselben mit Acetanhydrid in Gegenwart von Ameisensäureanhydrid zum 3-Nitro-4-oxo-2,6,7-trimethyl-4H-1-benzopyran vom Smp. 175-177° und Hydrierung desselben in Gegenwart von Palladium auf Calciumcarbonat in Dimethylformamid hergestellt werden.

Beispiel 29

In analoger Weise wie in Beispiel 2 beschrieben erhält man ausgehend von 3-Methoxyoxalylamino-4-oxo-2,6,7-trimethyl-4H-1-benzopyran das 3-Oxaloamino-4-oxo-2,6,7-trimethyl-4H-1-benzopyran vom Smp. über 200° (Zers.).

## Patentansprüche

1. 3-Oxaloamino-4-oxo-4H-1-benzopyranderivate

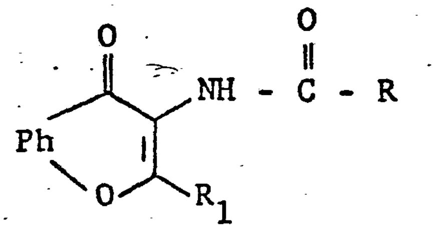

(I) ,

worin Ph gegebenenfalls substituiertes 1,2-Phenylen darstellt, R gegebenenfalls verestertes oder amidiertes Carboxy darstellt und $R_1$ Wasserstoff oder einen gegebenenfalls substituierten Kohlenwasserstoffrest bedeutet, in freier Form oder in Salzform,

2. Verbindungen gemäss Anspruch 1, worin R Carboxy, mit einem Alkohol aliphatischen Charakters verestertes Carboxy oder gegebenenfalls durch mindestens einen gegebenenfalls substituierten oder heteroanalogen Kohlenwasserstoffrest aliphatischen Charakters oder gegebenenfalls substituierten Arylrest substituiertes Carbamyl bedeutet, Ph gegebenenfalls ein- oder mehrfach durch aliphatische Reste, Acylreste, gegebenenfalls veräthertes oder verestertes Hydroxy und/oder Trifluormethyl substituiertes 1,2-Phenylen bedeutet und $R_1$ Wasserstoff, einen gegebenenfalls substituierten Kohlenwasserstoffrest aliphatischen Charakters oder aromatischen Kohlenwasserstoffrest bedeutet, wobei als Substituenten von aromatischen Gruppen jeweils Niederalkyl, Niederalkoxy, Halogen und Trifluormethyl in Betracht kommen.

3. Verbindungen gemäss Anspruch 1, worin R Carboxy, Niederalkoxycarbonyl oder gegebenenfalls durch Niederalkyl mono- oder disubstituiertes Carbamyl bedeutet, Ph gegebenenfalls durch Niederalkyl, 3- oder 4-gliedriges Niederalkylen, Hydroxy, Niederalkoxy, 2- oder 3-Hydroxyniederalkoxy, 3- oder 4-gliedriges Niederalkylendioxy, Niederalkanoyl und/oder Halogen, substituiertes 1,2-Phenylen bedeutet und $R_1$ Wasserstoff oder Niederalkyl bedeutet, jeweils in freier Form oder in Salzform.

4. Verbindungen gemäss Anspruch 1, worin R Carboxy oder Niederalkoxycarbonyl mit bis zu 5 C-Atomen bedeutet, Ph in einer der freien Stellungen gegebenenfalls durch Niederalkyl mit bis zu 4 C-Atomen, 3- oder 4-gliedriges Niederalkylen mit bis zu 4 C-Atomen, Niederalkoxy mit bis zu 4 C-Atomen, 3- oder 4-gliedriges Niederalkylendioxy, Niederalkanoyl mit bis zu 7 C-Atomen, Hydroxy und/oder Halogen bis Atomnummer 35 substituiertes 1,2-Phenylen bedeutet und $R_1$ Wasserstoff, Niederalkyl mit bis zu 4 C-Atomen oder Phenyl ist, in freier Form oder in Salzform.

5. Verbindungen der Formel Ia

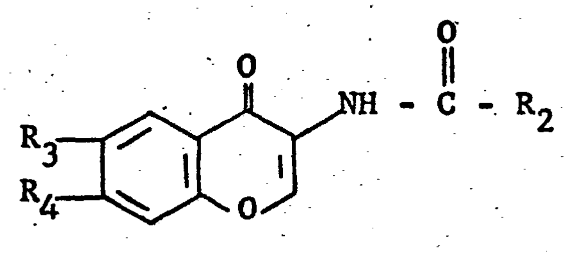 (Ia) ,

worin $R_2$ Carboxy bedeutet und $R_3$ und $R_4$ unabhängig voneinander Wasserstoff, Niederalkyl mit bis zu 4 C-Atomen, Niederalkoxy mit bis zu 4 C-Atomen, Niederalkanoyl mit bis zu 7 C-Atomen, Hydroxy oder Halogen bis Atomnummer 35 bedeuten oder gemeinsam einen 3- oder 4-gliedrigen Niederalkylen- oder Niederalkylendioxyrest mit bis zu 4 C-Atomen darstellen, in freier Form oder in Form eines pharmazeutisch verwendbaren Salzes.

6. Verbindungen gemäss Anspruch 5, worin $R_2$ Carboxy bedeutet und $R_3$ und $R_4$ Wasserstoff bedeuten oder worin $R_2$ Carboxy bedeutet, $R_3$ Wasserstoff ist und $R_4$ Hydroxy, Niederalkoxy mit bis zu 4 C-Atomen oder Halogen bis Atomnummer 35 bedeutet, in freier Form oder in Form eines pharmazeutisch verwendbaren Salzes.

7. Verbindungen gemäss Anspruch 5, worin $R_2$ Carboxy bedeutet und $R_3$ und $R_4$ unabhängig voneinander $C_1$-$C_4$-Alkyl oder gemeinsam $C_3$- oder $C_4$-Alkylen bedeuten, in freier Form oder in Salzform.

8. 3-Oxaloamino-4-oxo-4H-1-benzopyran oder ein pharmazeutisch verwendbares Salz davon.

9. 3-Methoxyoxalylamino-4-oxo-4H-1-benzopyran,
6-Hydroxy-3-methoxyoxalylamino-4-oxo-4H-1-benzopyran,
6-Hydroxy-3-oxoaloamino-4-oxo-4H-1-benzopyran oder ein pharmazeutisch verwendbares Salz davon,
6-Chlor-3-methoxyoxalylamino-4-oxo-4H-1-benzopyran,
6-Chlor-3-oxaloamino-4-oxo-4H-1-benzopyran oder ein pharmazeutisch verwendbares Salz davon,
6-Methoxy-3-methoxyoxalylamino-4-oxo-4H-1-benzopyran,
6-Methoxy-3-oxaloamino-4-oxo-4H-1-benzopyran oder ein pharmazeutisch verwendbares Salz davon,
5,8-Dimethyl-3-methoxyoxalylamino-4-oxo-4H-1-benzopyran,
5,8-Dimethyl-3-oxaloamino-4-oxo-4H-1-benzopyran oder ein pharmazeutisch verwendbares Salz davon,
3-Methoxyoxalylamino-6,7-trimethylen-4-oxo-4H-1-benzopyran oder
3-Oxaloamino-6,7-trimethylen-4-oxo-4H-1-benzopyran oder ein pharmazeutisch verwendbares Salz davon.

10. 5,7-Dimethyl-3-methoxyoxalylamino-4-oxo-4H-1-benzopyran,

3-Methoxyoxalylamino-8-methyl-4-oxo-4H-1-benzopyran,

6,7-Dimethyl-3-methoxyoxalylamino-4-oxo-4H-1-benzopyran,

3-Methoxyoxalylamino-6-methyl-4-oxo-4H-benzopyran,

6,7-Dimethyl-3-oxaloamino-4-oxo-4H-1-benzopyran oder ein pharmazeutisch verwendbares Salz davon,

6-Methyl-3-oxaloamino-4-oxo-4H-1-benzopyran oder ein pharmazeutisch verwendbares Salz davon,

6-Chlor-3-methoxyoxalylamino-7-methyl-4-oxo-4H-1-benzopyran,

6-Chlor-7-methyl-3-oxaloamino-4-oxo-4H-1-benzopyran oder ein pharmazeutisch verwendbares Salz davon,

6-Chlor-8-methyl-3-oxaloamino-4-oxo-4H-1-benzopyran oder ein pharmazeutisch verwendbares Salz davon,

6-Chlor-3-methoxyoxalylamino-8-methyl-4-oxo-4H-1-benzopyran oder ein pharmazeutisch verwendbares Salz davon,

N-(6,7-Dimethyl-4-oxo-4H-1-benzopyran-3-yl)-oxamid,

3-Methoxyoxalylamino-4-oxo-2,6,7-trimethyl-4H-1-benzopyran oder

3-Oxaloamino-4-oxo-2,6,7-trimethyl-4H-1-benzopyran oder ein pharmazeutisch verwendbares Salz davon.

11. 5,7-Dimethyl-3-oxaloamino-4-oxo-4H-1-benzopyran oder ein pharmazeutisch verwendbares Salz davon.

12. 3-Glykoloylamino-4-oxo-4H-1-benzopyranderivate der Formel

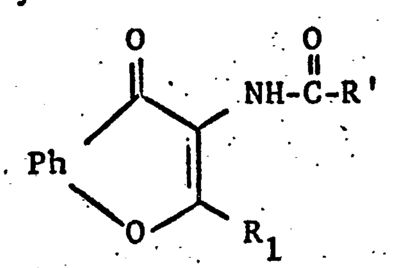

(I'),

worin Ph gegebenenfalls substituiertes 1,2-Phenylen darstellt, R' eine gegebenenfalls veresterte oder verätherte Hydroxymethylgruppe bedeutet und $R_1$ Wasserstoff oder einen gegebenenfalls substituierten aliphatischen Kohlenwasserstoffrest bedeutet, in freier Form oder in Salzform.

13. 3-Glykoloylamino-4-oxo-4,6,7,8-tetrahydro-cyclopenta[g]-1-benzopyran.

14. 3-Acetoxyglykoloylamino-4-oxo-4,6,7,8-tetrahydro-cyclopenta[g]-1-benzopyran.

15. Verfahren zur Herstellung neuer 3-Oxaloamino-4-oxo-4H-1-benzopyranderivate der Formel I

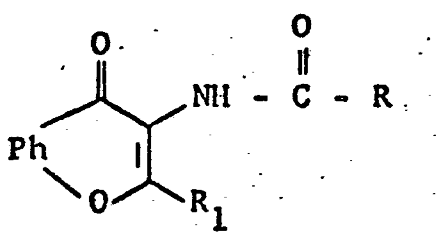

(I) ,

worin Ph gegebenenfalls substituiertes 1,2-Phenylen darstellt, R gegebenenfalls verestertes oder amidiertes Carboxy darstellt und $R_1$ Wasserstoff oder einen gegebenenfalls substituierten Kohlenwasserstoffrest bedeutet, in freier Form oder in Salzform, dadurch gekennzeichnet, dass man

a) eine Verbindung der allgemeinen Formel II

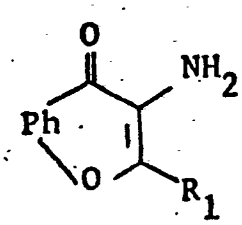

(II)

oder ein Säureadditionssalz davon mit einer Verbindung der Formel R-X (III) umsetzt, worin X eine gegebenenfalls funktionell abgewandelte Carboxygruppe bedeutet, oder

oder ein Säureadditionssalz davon mit einer Säure der Formel R'-COOH (IIIa) oder einem funktionellen Derivat davon umsetzt oder in einer Verbindung der Formel

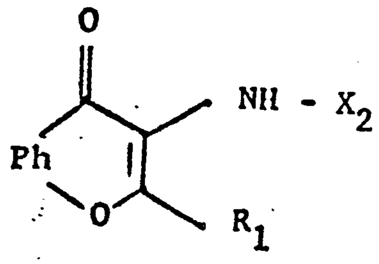

(IV'),

worin $X_2$ einen in die Gruppe R' überführbaren Rest bedeutet, oder in einem Salz davon, $X_2$ in die Gruppe R' überführt und gewünschtenfalls eine erhaltene Verbindung in eine andere Verbindung der Formel I' überführt und/oder eine erhaltene freie Verbindung in ein Salz oder ein erhaltenes Salz in die freie Verbindung oder in ein anderes Salz überführt.

17. Pharmazeutische Präparate enthaltend eine der in einem der Ansprüche 1 bis 14 beanspruchten Verbindungen in freier Form oder in Form eines pharmazeutisch verwenbaren Salzes.

18. Verwendung von in einem der Ansprüche 1-14 beanspruchten Verbindungen als Arzneimittel oder zur Herstellung eines solchen auf nicht-chemischem Wege.

19. Verbindungen gemäss einem der Ansprüche 1-14 als Arzneimittel.

b) in einer Verbindung der Formel

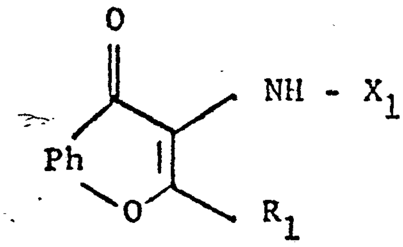

(IV)

worin $X_1$ einen in die gewünschte Gruppe der Formel R-C(=O)- überführbaren Rest bedeutet, $X_1$ in die Gruppe der Formel R-C(=O)- überführt und gewünschtenfalls eine so erhältliche Verbindung in eine andere Verbindung der Formel I umwandelt und/oder ein erhaltenes Salz in die freie Verbindung oder in ein anderes Salz oder eine erhaltene salzbildende Verbindung in ein Salz überführt.

16. Verfahren zur Herstellung neuer 3-Glykoloylamino-4-oxo-4H-1-benzopyranderivate der Formel

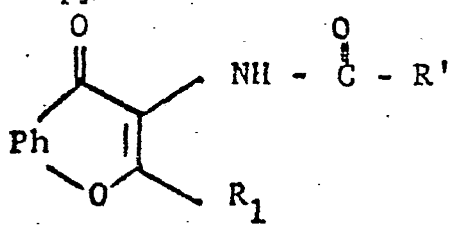

(I'),

worin Ph gegebenenfalls substituiertes 1,2-Phenylen bedeutet, R' eine gegebenenfalls veresterte oder verätherte Hydroxymethylgruppe darstellt und $R_1$ Wasserstoff oder einen gegebenenfalls substituierten Kohlenwasserstoffrest bedeutet, in freier Form oder in Salzform, dadurch gekennzeichnet, dass man eine Verbindung der Formel

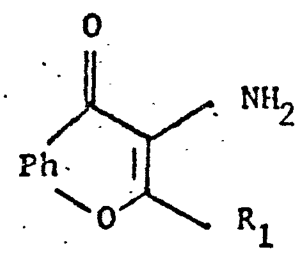

(II),

0000488

Europäisches Patentamt

# EUROPÄISCHER RECHERCHENBERICHT

Nummer der Anmeldung
EP 78 10 0327

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int.Cl.²) |
|---|---|---|---|
| | | | C 07 D 311/22<br>C 07 D 311/30<br>C 07 D 311/94<br>A 61 K 31/35 |
| D | US - A - 3 937 719 (J.H.SELLSTEDT, D.H.KLAUBERT)<br>* Spalten 1,3-6 *<br>---- | 1,8,15, 17-19 | |

RECHERCHIERTE SACHGEBIETE (Int. Cl.²)

C 07 D 311/22
C 07 D 311/30
C 07 D 311/94
A 61 K 31/35

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 23-10-1978 | FRANCOIS |